# EUROPÄISCHE PATENTSCHRIFT

(11) **EP 2 691 118 B1**
(45) Veröffentlichungstag und Bekanntmachung des Hinweises auf die Patenterteilung: **01.05.2019**
(21) Anmeldenummer: 12714607.4
(22) Anmeldetag: 29.03.2012
(51) Int. Cl.: C07H 21/02

(54) **PERFLUORIERTE VERBINDUNGEN ZUM NICHT-VIRALEN TRANSFER VON NUKLEINSÄUREN**
PERFLUORINATED COMPOUNDS FOR THE NON-VIRAL TRANSFER OF NUCLEIC ACIDS
COMPOSÉS PERFLUORÉS POUR LE TRANSFERT NON VIRAL D'ACIDES NUCLÉIQUES

(30) Priorität: 31.03.2011 DE 102011016334; 09.05.2011 DE 102011101361; 26.08.2011 DE 102011112191; 20.10.2011 DE 102011117390
(43) Veröffentlichungstag der Anmeldung: 05.02.2014
(73) Patentinhaber: Schäfer, Konstanze, 10405 Berlin (DE); Libera-Körner, Jeanette, 12487 Berlin (DE)
(72) Erfinder: Schäfer, Konstanze, 10405 Berlin (DE); Libera-Körner, Jeanette, 12487 Berlin (DE)
(74) Vertreter: Hertin und Partner Rechts- und Patentanwälte PartG mbB
(86) Internationale Anmeldenummer: PCT/EP2012/055639
(87) Internationale Veröffentlichungsnummer: WO 2012/130941

(56) Entgegenhaltungen:
- WO-A1-2005/116043
- WO-A2-2006/081035
- CHRISTIAN BELLER AND WILLI BANNWARTH: "Noncovalent Attachment of Nucleotides by Fluorous-Fluorous Interactions: Application to a Simple Purification Principle for Synthetic DNA Fragments", HELVETICA CHIMICA ACTA, VERLAG HELVETICA CHIMICA ACTA, BASEL, CH, Bd. 88, 1. Januar 2005 (2005-01-01), Seiten 171-179, XP002478874, ISSN: 0018-019X, DOI: 10.1002/HLCA.200490291
- WILLIAM H PEARSON ET AL: "Fluorous Affinity Purification of Oligonucleotides", JOURNAL OF ORGANIC CHEMISTRY, ACS, US, Bd. 70, Nr. 18, 1. Januar 2005 (2005-01-01), Seiten 7114-7122, XP002478873, ISSN: 0022-3263, DOI: 10.1021/JO050795Y [gefunden am 2005-08-02]
- Izabela Tworowska ET AL: "Synthesis of Tri- and Tetracoordinate Phosphorus Compounds Containing a PCF3 Group by Nucleophilic Trifluoromethylation of the Corresponding PF Compounds", Angewandte Chemie International Edition, vol. 40, no. 15, 3 August 2001 (2001-08-03), pages 2898-2900, XP055472233, ISSN: 1433-7851, DOI: 10.1002/1521-3773(20010803)40:15<2898::AID -ANIE2898>3.0.CO;2-I
- Andrzej Wilk ET AL: "The 4-[ N -Methyl- N -(2,2,2-trifluoroacetyl)amino]butyl Group as an Alternative to the 2-Cyanoethyl Group for Phosphate Protection in the Synthesis of Oligodeoxyribonucleotides", JOURNAL OF ORGANIC CHEMISTRY, vol. 64, no. 20, 1 October 1999 (1999-10-01), pages 7515-7522, XP055282235, ISSN: 0022-3263, DOI: 10.1021/jo990835w
- Cristina Ausín ET AL: "Assessment of heat-sensitive thiophosphate protecting groups in the development of thermolytic DNA oligonucleotide prodrugs", TETRAHEDRON, vol. 66, no. 1, 1 January 2010 (2010-01-01), pages 68-79, XP055472212, AMSTERDAM, NL ISSN: 0040-4020, DOI: 10.1016/j.tet.2009.10.096
- William H. Connors ET AL: "Synthesis of Oligonucleotides with a 2'-Cap at the 3'-Terminus via Reversed Phosphoramidites", ORGANIC LETTERS , 14(23), 6012-6015 CODEN: ORLEF7; ISSN: 1523-7052, vol. 5, no. 3, 1 February 2003 (2003-02-01), pages 247-250, XP055472745, ISSN: 1523-7060, DOI: 10.1021/ol020212w
- Ramon Eritja ET AL: "O-aryl phosphoramidites: synthesis, reactivity and evaluation of their use for solid-phase synthesis of oligonucleotides", TETRAHEDRON, vol. 46, no. 3, 1 January 1990 (1990-01-01), pages 721-730, XP055472767, AMSTERDAM, NL ISSN: 0040-4020, DOI: 10.1016/S0040-4020(01)81356-9
- GAO H ET AL: "H-PHOSPHONATE OLIGONUCLEOTIDE SYNTHESIS ON A POLYETHYLENE GLYCOL/POLYSTYRENE COPOLYMER", TETRAHEDRON LETTERS, ELSEVIER, AMSTERDAM, NL, vol. 32, no. 40, 1 January 1991 (1991-01-01), pages 5477-5479, XP000651238, ISSN: 0040-4039, DOI: 10.1016/0040-4039(91)80062-B
- William H. Connors ET AL: "Supporting Information: Synthesis of Oligonucleotides with a 2'-Cap at the 3'-Terminus via Reversed Phosphoramidites", ORGANIC LETTERS , 14(23), 6012-6015 CODEN: ORLEF7; ISSN: 1523-7052, vol. 5, no. 3, 1 February 2003 (2003-02-01), pages 247-250, XP055472810, ISSN: 1523-7060, DOI: 10.1021/ol020212w & WILLIAM H. CONNORS ET AL: "Supporting Information: Synthesis of Oligonucleotides with a 2'-Cap at the 3'-Terminus via Reversed Phosphoramidites", ORGANIC LETTERS , 14(23), 6012-6015 CODEN: ORLEF7; ISSN: 1523-7052, vol. 5, no. 3, 1 February 2003 (2003-02-01), pages 247-250, XP055472813, ISSN: 1523-7060, DOI: 10.1021/ol020212w

## Beschreibung

Gegenstand der vorliegenden Erfindung ist eine Verbindung gemäß Anspruch 1, deren Verwendung gemäß den Ansprüchen 4 und 5 und eine pharmazeutische Zusammensetzung gemäß Anspruch 6.

Nicht-viraler Gentransfer ist ein wichtiger Forschungsschwerpunkt in der Grundlagenforschung und in der Medizin. Anwendungsmöglichkeiten ergeben sich insbesondere bei klassischen Erbkrankheiten und bei erworbenen genetischen Erkrankungen (z.B. HIV, chronische Infektionskrankheiten, Tumore, Herz- und Kreislaufkrankheiten). Versuche, die Gentherapie in der Medizin zu etablieren, wurden in der Vergangenheit vor allem mittels viraler Vektoren vorgenommen. Diese zeigen aber erhebliche Nachteile. Die Anwendungen sind nur unzureichend sicher und lösen außerdem nach einmaliger Anwendung im Körper Immunreaktionen aus, die eine zweite Applikation unmöglich machen. Abgesehen davon wurde immer wieder von Zwischenfällen berichtet, bei denen Patienten infolge der Behandlung schwer erkrankten oder sogar verstarben.

Eine Alternative zum viralen Gentransfer könnte der nicht virale Gentransfer sein. Aber alle bisher bekannten Methoden sind so uneffizient, dass sie in der Medizin keine Anwendung finden. Zu den nicht viralen Transfermethoden zählen alle Verfahren, in die keine Viren involviert sind.

Ein Transfer nackter DNA oder RNA wurde bereits erforscht, bietet aber praktisch gesehen in der bisherigen Form wenig Anwendungsmöglichkeiten, da man auf offenes Gewebe transfiziert oder in die Blutbahn injiziert und RNA und DNA sehr anfällig gegenüber Nukleasen sind. Außerdem sind die Transfektionsraten sehr gering.

Um die oben genannten Probleme zu umgehen, gewinnt der nicht virale Nukleinsäuretransfer mittels Komplexen aus DNA oder RNA mit kationischen Polymeren (z.B. PEI, PEG, PLL, PLA) oder mit kationischen Lipiden (z.B. CTAB, DOTMA, DOTAP) zunehmend an Bedeutung. Hierbei nutzt man die positive Ladung solcher Moleküle, um die negative Ladung des Zucker-Phoshat-Gerüstes der Nukleinsäuren zu neutralisieren und eine Aufnahme durch die Zellmembran ins Zytoplasma der Zelle zu erleichtern. Zu diesen Methoden gibt es eine Reihe von Patenten. Diesbezügliche Forschungsergebnisse markieren aber erst den Beginn einer Entwicklung. Denn abgesehen von den noch unbefriedigenden Transfektonsraten stellt die Toxizität dieser Polymere und Lipide für die Zelle eine entscheidende Hürde dar. Abgesehen davon neigen diese Komplexe innerhalb des Cytoplasmas zu Verklumpungen, da die biologische Abbaubarkeit des Polymers zu gering ist. Die Beladungraten mit DNA oder RNA steigen mit der Höhe der positiven Ladung des Polymers oder des Lipids. Aber genau diese stark positiv geladenen Moleküle haben sich als besonders toxisch für Zellen herausgestellt. Um die Toxizität dieser kationischen Polymere und Lipide herabzusetzen, kombiniert man diese zunehmend mit hydrophilen Polymeren, was aber keine herausragende Verbesserung bringt.

Bisher bekannte Transportmoleküle für den nicht-viralen Gentransfer haben neben ihrer geringen Effizienz einen gemeinsamen zweiten Nachteil: sie verbleiben nach dem Transport in die Zelle im Cytoplasma und akkumulieren dort oder reagieren mit Zellmolekülen oder sie haben negative Auswirkungen auf die Zellmembran.

Darüber hinaus wird daran geforscht, Nukleinsäurebaustene so zu modifizieren, dass diese für einen nicht viralen Transfer von Nukleinsäuren geeignet sind. So werden in WO 2008/039254 und Patent US 2010/0016409 RNA-Partikel beschrieben, die teilweise oder ganz doppelsträngig oder in anderen bestimmten Konformationen vorliegen und gegebenenfalls mit anderen Molekülen verknüpft sind. Diese RNA-Partikel, die aufgrund ihrer Konformation eine höhere Stabilität als einzelsträngige mRNA aufweisen, werden für den nicht viralen Gentransfer vorgeschlagen. Der Vorteil dieser Moleküle liegt darin, dass diese sehr klein sind und auch sehr feine kapillare Blutgefässe passieren können. Außerdem besteht dadurch kaum eine Verklumpungsgefahr, wie sie bei relativ großen Polymerkomplexen oft auftritt. Ein Nachteil dieser Moleküle ist, dass zu den therapeutischen Sequenzen zusätzliche Sequenzen in das mRNA-Molekül eingebaut werden müssen, die zur Selbstzusammenlagerung bestimmter Bereiche führen sollen, infolgedessen solche RNA-Konformationen wie Haarnadel-, Nanoring-, Quadratstruktur und andere entstehen. Obwohl die RNA-Moleküle eine längere Lebensdauer innerhalb des Organismus besitzen als reine einzelsträngige mRNA, ist die Verfügbarkeit dieser doppelsträngigen Konformationen zur Translation an den Ribosomen noch nicht erwiesen.

In EP 1 800 697 B1 wird eine modifizierte mRNA beschrieben, deren G/C-Gehalt verglichen zum Wildtyp erhöht ist und dass mindestens ein Codon der Wildtyp-Sequenz, das für eine in der Zelle relativ seltene tRNA codiert, gegen ein Codon ausgetauscht ist, das für eine in der Zelle relativ häufige tRNA codiert. Diese so modifizierte mRNA ist zusätzlich so verändert, dass mindestens ein Nukleotid-Analoge aus der Gruppe, bestehend aus Phosphorthioaten, Phosphoramidaten, Peptidnucleotiden, Methylphosphonaten, 7-Deazaguanosin, 5-Methylcytosin und Inosin eingebaut wird, die bereits in einigen anderen RNA-Verfahren (siRNA) Anwendung fanden. Das Verfahren ist beschrieben für sequenzveränderte mRNAs von ursprünglichen Wildtyp-Peptiden.

Dokument WO 99/14346 beschreibt ebenfalls eine stabilisierte mRNA durch Sequenzmodifikationen, insbesondere die Verminderung des C-und/oder U-Gehalts durch Baseneliminierung oder Basensubstitution.

Die Patente US 5,580,859 und US 6,214,804 beschreiben transiente Gentherapie-Konstrukte, die einen Expressionsvektor erfordern und von DNA-Konstrukten ausgehen.

WO 02/098443 beschreibt mRNAs, die für ein biologisch wirksames Peptid codieren, welches in dem zu behandelnden Patienten nicht oder nur unzureichend fehlerhaft gebildet wird, um eine Immunantwort auszulösen.

Eine weitere Entwicklung auf dem Gebiet der nicht viralen Gentherapie ist die "Microbubble"-Technologie, in der mit Nukleinsäure gefüllte stabilisierte Protein-Mikrosphären (Kausik Sarkara et al., J. Acoust. Soc. Am. 118, 1, July 2005, Pages: 539-550) oder Zucker-Mikrosphären (Schlief et al., Ultrasound in Medicine & Biology, Volume 22, Issue 4, 1996, Pages 453-462) zusätzlich mit Ultraschallgasen befüllt wurden. Es hatte sich gezeigt, dass Ultraschall-Kontrastmittel zur Verstärkung der Kavitation führen, wodurch die Zellmembran transient permeabilisiert wird (Tachibana et al., Echocardiography. 2001 May;18(4):323-8. Review). Dies führte zu einer gesteigerten Aufnahme der nicht viralen Gentransfer-Konstrukte in die Zelle. Trotzdem wurde nicht die Effizienz des viralen Gentransfers erreicht.

Die Ultraschallmethode mit Kontrastmittel wird zunehmend auch verwendet, um die Effizienz des viralen Gentransfers zu steigern (Blomley, 09/2003, Radiology, 229, 297-298).

Als besonders geeignet für die "Microbubble"-Technologie haben sich neben einer Reihe von anderen Gasen Perfluorocarbon-Gase gezeigt. Durch ihre stark lipophilen Eigenschaften und ihre extrem niedrige Oberflächenspannung sind sie bestens geeignet, die Integrität der Zellmembran zu stören und einen Durchtritt von Stoffen zu ermöglichen. Hier handelt es sich um reine Perfluorocarbone, die nicht auf irgendeine Weise mit anderen Bestandteilen gebunden sind. Experimente zum nicht viralen Gentransfer mittels reiner Perfluorocarbone haben aber gezeigt, dass die Nukleinsäuren von den reinen Perfluorocarbonen abdiffundieren, bevor deren Eintritt in die Zelle erfolgt ist. Auf diese Weise können Nukleinsäuren nur infolge von Zufallsereignissen mitgerissen werden.

Alle bisherigen Lösungen sind noch weit von der Effizienz eines viralen Gentransfers entfernt. Es besteht ein großes Interesse an der Entwicklung eines nicht-viralen Transfersystems für Nukleinsäuren in die Zelle, dessen Effektivität einerseits mindestens gleichwertig zur Effektivität des viralen Gentransfers ist und dessen Bestandteile andererseits nicht in der Zelle akkumulieren, nicht mit den Zellmolekülen reagieren und nicht schädlich auf die Zellmembran wirken.

Perfluorocarbone (PFC) sind als chemische Stoffe bekannt und die strukturelle Modifizierung von Nukleotiden mit PFCs ist im Stand der Technik beschrieben (WO 2006/081035 A2, Beller und Bannwarth, Helvetica Chimica Acta, Bd. 88, 2005, 171-179, Pearson et al, J. of Organic Chemistry, Bd. 70, Nr. 18, 2005, 7114-7122, WO 2005/116043 A1, Tworowska et al, Angewandte Chemie International Edition, Bd. 40, Nr. 15, 2001, 2898-2900, Wilk et al, J. of Organic Chemistry, Bd. 64, Nr. 20, 1999, 7515-7522, Ausin et al, Tetrahedron, Bd. 66, Nr. 1, 2010, 68-79, Connors et al, Organic Letters, 14(23), 6012-6015, Eritja et al, Tetrahedron, Bd. 46, Nr. 3, 1990, 721-730, Gao et al, Tetrahedron Letters, Bd. 32, Nr. 40, 1991, 5477-5479).

Entsprechend besteht eine Aufgabe der vorliegenden Erfindung darin eine stabile Verbindung bereitzustellen, die die Nachteile des viralen Gentransfers überwindet und für den nicht-viralen Gentransfer geeignet ist.

Die vorliegende Aufgabe wurde entsprechend durch die Bereitstellung einer Verbindung für den nicht-viralen Transfer von Nukleotidbausteinen mit den Merkmalen des Anspruchs 1 gelöst.

Die Erfindung betrifft eine Verbindung der allgemeinen Formel (XIX)

A - B - C(F', G')- B- E (XIX)

oder der allgemeinen Formel (XXII)

A - B - C(F', G')- E (XXII)

wobei
- A ein Perfluorocarbon (PFC) ist,
- B mindestens eine Sollbruchstelle ist,
- C abwesend oder ein Linker ist, wobei der Linker ausgewählt ist aus der Gruppe bestehend aus: geradlinige oder verzweigte acyclische oder cyclische, polycyclische oder heterocyclische aliphatische Alkane, Alkene, Alkine, aromatische Verbindungen,
- E ein Oligonukleotid oder eine Nukleinsäure ist,
- F' abwesend oder ein Ligand ist, wobei der Ligand ausgewählt ist aus der Gruppe bestehend aus: Transferrin, Folsäure, Galactose, Lactose, Mannose, Epidermal Growth Factor, RGD-Peptide, Biotin oder einer Kernlokalisationssequenz,
- G' abwesend oder ein Marker ist, wobei der Marker einen Fluoreszenz-Farbstoff ist,
wobei:
- A, B, C, E, F',G' über kovalente Bindungen verknüpft sind, und
- A-B, optional mit C, F' und/oder G', an E an der 2'- Position der Ribose, an der Phosphatgruppe oder an der Nukleobase verknüpft ist,
wobei:
A ausgewählt ist aus der Gruppe der Perfluorkohlenwasserstoffe (PFC) bestehend aus C4-C30, höchst bevorzugt C4-C20, Alkane, Alkene oder Alkine, die linear oder verzweigt sein können, bei denen alle H-Atome durch F-Atome ersetzt sind,
und wobei:
die Sollbruchstelle B in Form einer glykosidischen Bindung, einer Disulfidbrücke, einer Estergruppe, Ethergruppe, Peptidbindung, Imin-Bindung, Hydrazon-Bindung, Acylhydrazon-Bindung, Ketalbindung, Acetalbindung, cis-Aconitrilbindung, Tritylbindung, beta-D-Glucosylceramid und/oder Dithiothreitol, oder in Form eines Plasmalogenperfluorids, einer Vinylethergruppe oder Orthoesters ausgebildet ist.

Eine neue, vielversprechende Stoffgruppe für den nicht viralen Gentransfer sind demnach Molekülverbindungen aus insbesondere Perfluorocarbonen (PFCs) und z.B. Nukleinsäurebausteinen, die über eine Sollbruchstelle miteinander verbunden sind, sog. perfluorierte Nukleinsäurebausteine.

Die Vorteile von perfluorierten Nukleinsäurebausteinen sind folgende:
1) Durch ihre unter physiologischen Bedingungen starke lipophile Wirkung (lipophiler als Fettsäuren) und noch mehr durch ihre extrem niedrige Oberflächenspannung lagern sich diese Moleküle schnell auf der Oberfläche der Zellmembran an. Dort werden sie durch regulär stattfindende Prozesse wie Pinozytose, Phagozytose, Endocytose oder Endozytoseunabhängige Wege in die Zelle aufgenommen. Dabei sind die PFCs das Transportsystem für die sonst nur schwer aufnehmbaren Nukleinsäurebausteine in die Zelle.
2) PFCs sind aufgrund ihrer starken C-F - Bindung sehr innert und reagieren nicht mit Zellmolekülen.
3) PFCs sind nach ihrer Abspaltung von den Nukleinsäurebausteinen kleine, ungeladene lipophile Moleküle, die entsprechend dem Konzentrationsgradienten passiv aus der Zelle entweichen können.
4) Durch die medizinische Anwendung von PFCs als Sauerstoffträger/Blutersatzstoff beim Menschen konnte gezeigt werden, dass PFCs aus dem Körper über Lunge, Nieren und Haut ausgeschieden werden.
5) Durch die bereits erfolgte medizinische Zulassung als Blutersatzstoff und als Kontrastmittel erscheint eine medizinische Zulassung von PFCs für den nicht-viralen Gentransfer einfacher.
6) Perfluorierte Nukleinsäure zeigen eine signifikant höhere Aufnahme in die Zelle als andere Stoffe des nicht-viralen Gentransfers und sind eine echte Alternative zum viralen Gentransfer.
7) Im Gegensatz zum viralen Gentransfer erzeugen perfluorierte Nukleinsäuren keine Immunantwort des Körpers und können beliebig oft eingesetzt werden.
8) In Verbindung mit einem mRNA-Transfer kann aufgrund der begrenzten Lebensdauer der mRNA und der unbegrenzten Wiederholbarkeit des Transfers eine Dosierbarkeit erzielt werden.

Bisher genutzte reine Perfluorocarbone sind ursprünglich in der Industrie als Hochleistugsschmierstoffe bekannt. Auf pharmazeutischem Gebiet wurden sie bisher vor allem aufgrund ihrer hohen Sauerstofflöslichkeit als Blutersatzstoffe oder auch als Kontrastmittel verwendet. Auch konnte gezeigt werden, dass in Ultraschall-Anwendungen beim nicht viralen Gentransfer Microbubbles, die mit gasförmigen reinen Perfluorocarbonen gefüllt waren, im Vergleich zu anderen Gasen die Effizienz des Gentransfers steigern konnten.

Für den nicht-viralen Transfer von Nukleinsäuren in die Zelle sind reine Perfluorocarbone aber kaum geeignet, da Nukleinsäuren nicht an ihnen haften und somit nur durch Zufallsereignisse mitgerissen werden. Es bedarf einer echten Bindung zwischen Perfluorocarbonen und Nukleinsäurebausteinen, die durch eine Sollbruchstelle spaltbar ist.

Die erfindungsgemäße Zusammensetzung ist demnach gekennzeichnet durch die Aufnahme der perfluorierten Nukleinsäure in die Zelle, dem Bruch der Sollbruchstelle zwischen Nukleinsäure und Perfluorcarbonmolekülen, der Freisetzung der Spaltprodukte (Nukleinsäure einerseits und Perfluorocarbon-Moleküle andererseits) ins Cytoplasma und der anschließenden Diffusion oder der aktiven Ausschleusung der Perfluorocarbon-Moleküle aus der Zelle. Eine Endocytose-unabhängige Aufnahme der perfluorierten Nukleinsäure ist ebenfalls möglich.

Wie oben ausgeführt sind perfluorierte Nukleinsäuren sowohl geladene als auch sehr lipophile Moleküle. Die Sekundär- und Tertiarstruktur dieser Moleküle ist sehr geeignet, die Zellmembran zu destabilisieren und in die Zelle aufgenommen zu werden. Perfluorocarbone sind unter physiologischen Bedingungen noch lipohpiler als Fettsäuren und haben eine extrem niedrige Oberflächenspannung, die es dem Molekül ermöglicht, sich großflächig auf der Zellmembran auszudehnen.

Durch eine Sollbruchstelle zwischen Nukleinsäurebaustein und perfluoriertem Teil des Moleküls nach Eintritt in die Zelle wird der perfluorierte Teil von der Nukleinsäure abgespalten. Dies geschieht in der Regel durch säurelabile Sollbruchstellen. Das erhöhte Reduktionspotential im Cytoplasma und mehr noch der niedrige pH-Wert in den Endosomen (bis zu pH = 4,5) schaffen die Voraussetzung zu deren Hydrolyse.

Die Sollbruchstellen für dieses System sind so ausgesucht, dass die Spaltprodukte möglichst keine oder wenig Molekülveränderungen erfahren. Sehr geeignet hierfür sind "spurlose" Sollbruchstellen, die eine unveränderte Nukleinsäure hinterlassen und einen Perfluorocarbonrest, der seine extrem lipophile und unpolare Natur erhalten hat. Ein Beispiel für eine derartige Sollbruchstelle ist in dem folgenden Schema 1 dargestellt:

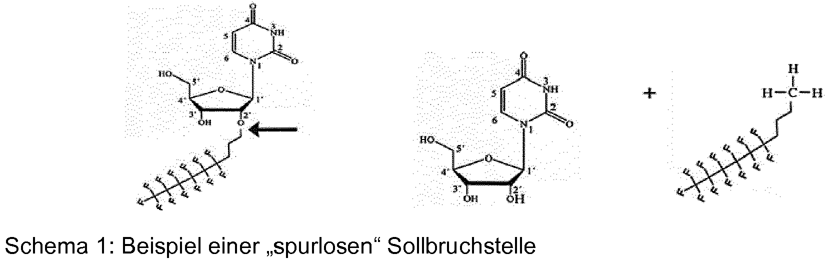

Die ins Cytoplasma freigesetzten Nukleinsäuren sind für die Zelle frei zugänglich. Sie können ihren Wirkungsort im Cytoplasma besitzen wie z.B. mRNA, siRNA, microRNA, Aptamere, Antisense-RNA und andere, oder sie können in den Nukleus transportiert werden wie z.B. DNA mit oder ohne Kernlokalisationssequenz oder Antisense-Oligonukleotide.

Die ebenfalls ins Cytoplasma entlassenen Perfluorocarbon-Moleküle sind bei entsprechender Sollbruchstelle ungeladen, lipophil und sehr klein. Diese Eigenschaften sind die Voraussetzung für eine freie Diffusion entlang des Konzentrationsgradienten durch die Zellmembran hindurch. Auch möglich ist Exocytose oder ein anderer Ausscheideweg aus der Zelle. Perfluorocarbon-Moleküle sind extrem innert und reagieren nicht mit Zellmolekülen. Solange sie relativ unverändert in ihrer Molekülstruktur vorliegen, lagern sie sich auch nicht in Lipiden ein. Durch die medizinische Anwendung von Perfluorcarbonen als Blutersatzstoffe ist bekannt, dass sie aus dem Körper über Lungen- und Nierenfunktion sowie über die Haut ausgeschieden werden.

Die perfluorierten Nukleinsäuren können zusätzlich mit Fluoreszenzfarbstoffen verlinkt sein, um deren Weg in der Zelle nach zu verfolgen. Durch eine Verlinkung mit spezifischen Liganden oder anderen Erkennungssequenzen kann das System für eine Behandlung spezieller Zelltypen eingestellt werden. Prinzipiell ist das Transfersystem aus perfluorierten Nukleinsäuren für jede Anwendung nutzbar, in der Nukleinsäuren oder modifizierte Nukleinsäuren-Analoge in eine Zelle transportiert werden sollen.

Typischerweise können im Rahmen der vorliegenden Erfindung als A Moleküle bzw. A Molekülreste ausgewählt aus der PFC-Gruppe enthaltend -(CₙF₍₂ₙ₊₂₎₋₁) mit n ≥ 4, bevorzugt n = 4-30, wie z.B. -C₄F₉, -C₄F₁₁ usw., -(CₙF₂ₙ₋₁) mit n ≥ 4, bevorzugt n = 4-20 wie z.B. -C₄F₇ usw., -(-CₙF₍₂ₙ₋₂₎₋₁) mit n ≥ 4, bevorzugt n = 4-20, wie z.B. -C₄F₅, -C₅F₇ usw. verwendet werden.

Zur Herstellung der erfindungsgemäßen Verbindungen ist es sinnvoll, perfluorierte Verbindungen zu verwenden, die über eine geeignete Funktionalität verfügen, um eine kovalente Bindung mit den weiteren Molekülen wie z.B. B und E eingehen zu können. Diese Funktionalität der als Ausgangssubstanz verwendeten perfluorierten Verbindung ermöglicht insbesondere eine Addition, Substitution, Veresterung, Veretherung, Kondensation etc. Derartige Verknüpfungsreaktionen sind dem Fachmann bekannt. Bevorzugte Funktionalitäten sind ausgewählt sind aus der Gruppe enthaltend Halogenalkane, Hydroxyl-, Ether-, Amino-, Sulhydryl-, Aldehyd- Keto-, Carboxyl-, Ester- und Säureamidgruppen, Gruppen mit Radikalen oder Ionen und Moleküle aus den Stoffgruppen der Carbonsäuren, Peroxycarbonsäuren, Thiocarbonsäuren, Sulfonsäuren, Sulfinsäuren, Sulfensäuren, Sulfoxiden, Carbonsäuresalzen, Sulfonsäuresalzen, Sulfinsäuresalzen, Sulfensäuresalzen, Carbonsäureanhydriden, Carbonsäureestern, Sulfonsäureestern, Carbonsäurehalogeniden, Sulfonsäurehalogeniden, Carbonsäureamiden, Sulfonsäureamiden, Carbonsäurehydraziden, Nitrilen, Aldehyden, Thioaldehyden, Ketonen, Thioketonen, Oximen, Alkoholen, Phenolen, Thiolen, Aminen, Iminen, Hydrazinen, Ethern, Estern, Thioethern, Thioestern, Halogenwasserstoffen, Nitroverbindungen, Nitrosoverbindungen, Azoverbindungen, Diazoverbindungen, Diazoniumsalzen, Isocyanaten, Cyanaten, Isocyaniden, Thiocyanaten, Isothiocyanaten, Hydroperoxiden, Peroxiden, 2'-Carbamate, Ether, Säureamide und Thioether oder Verbindungen, die aufgrund ihrer Mehrfachbindung reaktiv sein können. Besonders bevorzugt sind Heteroatome wie Br, I, Cl, H, Si, N, O, S, P, Hydroxyl-, Amino-, Carboxylgruppen, Halogenalkane, Carbonsäureamiden, Alkoholen, Hydrazinen, Isocyanaten, Thiocyanaten und Säureamide.

Bevorzugterweise wird als Startverbindung für die Bereitstellung des Moleküls A in der Verbindung der Erfindung eine Substanz verwendet, die zur nukleophilen Substitution geeignet ist. Die Startsubstanz zur Bereitstellung des Moleküls A weist eine nukleophile Abgangsgruppe auf, die für einen Fachmann ohne weiteres erkennbar sind.

A Moleküle sind hiermit offenbart, sie auf einer der folgenden Startsubstanzen basieren:
- F(CF₂)ₙX, wobei n= 1-50, bevorzugt n = 1-10, und X = Br, I, Cl, H oder X= Si, N, O, S, P, in Verbindung mit einer funktionellen Gruppe, insbesondere C₈F₁₇I, C₈F₁₇Br;
- F(CF₂)ₙ-(CH₂)ₘX, wobei n = 1-50, bevorzugt n = 1-10, m = 1-26, bevorzugt m = 1 - 6 und X = Si, N, O, S, P, Br, I, H;
- F(CF₂)n-O_{b}-CH=CH₂ mit n = 1-50, bevorzugt n = 1-10, und b = 0 oder 1, bevorzugt b = 0;
- C₆F₁₃CH₂CH₂Mgl, (C₆F₁₃CH₂CH₂)₃SnPh, (C₆F₁₃CH₂CH₂)₃SnBr, (C₆F₁₃CH₂CH₂)₃SnH;
- C₂F₅I, C₃F₇Br, C₄F₉I, C₅F₁₁Br, C₆F₁₃Br, C₈F₁₅Br, C₁₀F₁₇I,
- C₄F₉CH=CHC₄F₉, C₈F₁₆Cₗ₂, C₁₀F₁₉N, C₆F₁₉Br, C₉F₂₁N, C₁₀F₂₁Br, C₁₁F₂₂N₂O₂, C₆F₁₃CH=CHC₆F₁₃, C₁₂F₂₇N, C₁₆F₂₅Br;
- C₈F₁₇I, C₈F₁₇Br.

Weitere Ausgangssubstanzen der A-Moleküle aus der Gruppe der Perfluorkohlenwasserstoffe sind hiermit offenbart:
- perfluorierte Cholesteryl- und Adamantylverbindungen, perfluorierte cis-Eicosenoic, perfluorierte aromatische Verbindungen, perfluorierte Pyrene, perfluorierte Glyceride; und 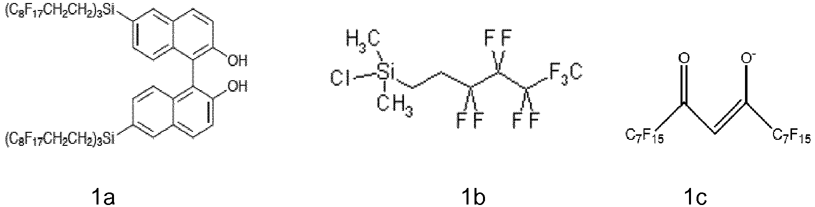 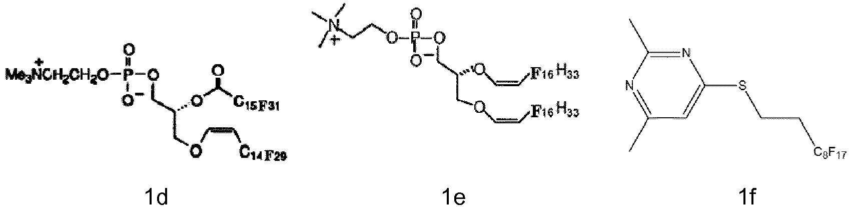 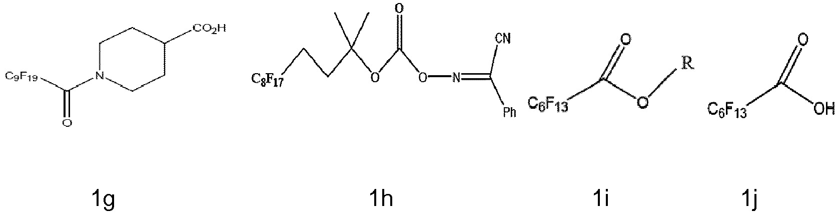 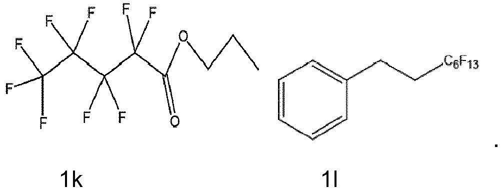
- zur Verknüpfung in Form einer lonenbindung können z.B. folgende PFC's verwendet werden: 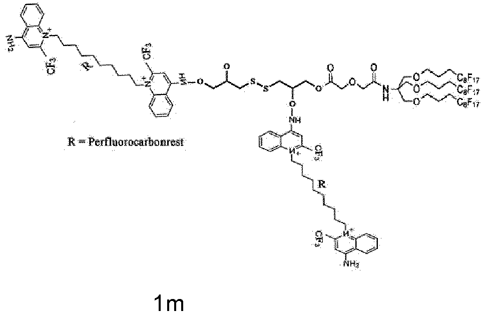 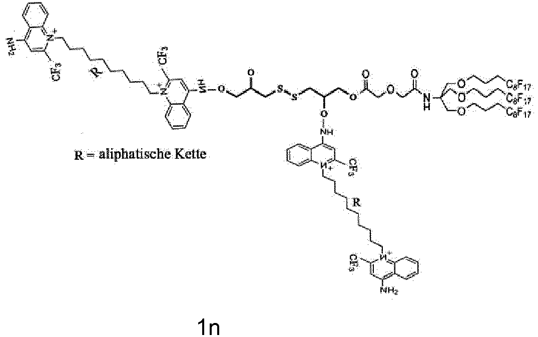 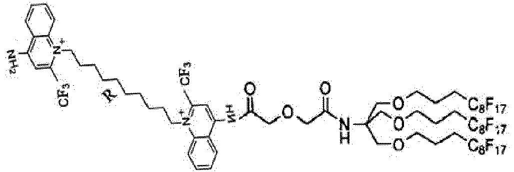 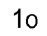 mit R = Perfluorocarbonrest oder aliphatische Kette;

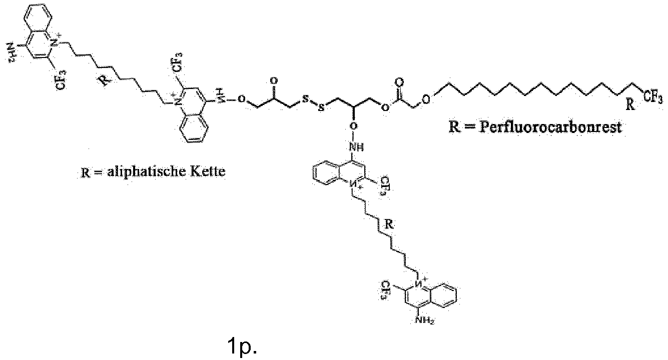

Es ist ebenfalls bevorzugt, dass die vorliegende Verbindung zwei und mehr Moleküle A ausgewählt aus der Gruppe der Perfluorocarbone (PFC) enthält.

Typischerweise können die oben beschriebenen funktionalisierten perfluorierten Verbindungen zu mehreren Einheiten zusammengefasst werden. Als Beispiele sei auf die folgenden Moleküle hingewiesen:

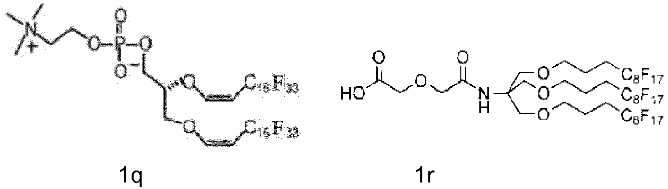

Die Anbindung dieser Moleküle an die weiteren in der Verbindung enthaltenen Moleküle kann über NH₂-Gruppen oder OH-Gruppen oder andere geeignete Gruppen erfolgen.

Gemäß Anspruch 1 ist die mindestens eine Sollbruchstelle B in Form einer glykosidischen Bindung, einer Disulfidbrücke, einer Estergruppe, Ethergruppe, Peptidbindung, Imin-Bindung, Hydrazon-Bindung, Acylhydrazon-Bindung, Ketalbindung, Acetalbindung, cis-Aconitrilbindung, Tritylbindung, beta-D-Glucosylceramid, und/oder Dithiothreitol, oder in Form eines Plasmalogenperfluorids, einer Vinylethergruppe oder Orthoesters.

Sollbruchstellen besitzen innerhalb der Konstrukte von PFCs und Nukleinsäure zwei wichtige Funktionen. Zum einem dienen die Sollbruchstellen dem sogenannten "Leakage". Hierbei lagern sich die Partikel der PFC-Nukleinsäure-Verbindungen in die Endosomen-Membran von Zellen ein und müssen anschließend aus diesen Endosomen freigesetzt werden. Das geschieht durch die Zerstörung der Integrität der Membran, welche durch die Spaltung der Konstrukte hervorgerufen wird. Zum anderen muss der Rest des PFC-Moleküls abgespalten werden, um die Nuleinsäure für die Zelle verfügbar zu machen. Hierbei werden die säurelabilen Sollbruchstellen wie die oben angeführten glycosidischen Bindungen, DisulfidBrücken, Ester oder Ether z.B. an der 2'-Position des Nukleotids oder an anderen Stellen, unter sauren Bedingungen chemisch oder durch Hydrolasen/Esterasen hydrolysiert (gespalten).

Komplexere Sollbruchstellen können in Form von spezifischen Substraten für Enzyme, pH- und photo-sensitive Lipidfunktionen, Molekülen mit Freisetzung durch Ultraschalleinwirkung oder temperaturgesteuerten Lipidmodifizierungen sowie anderen unter physiologischen Bedingungen spaltbarem Bindungen vorliegen.

Beispielhaft sei auf die folgenden Sollbruchstellen hingewiesen:
- Plasmalogenperfluorid, spaltbar durch Licht 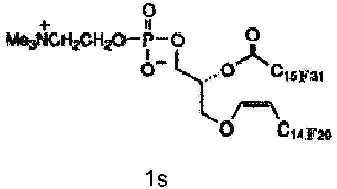
- pH-sensitive perfluorierte Vinyletherfunktionen 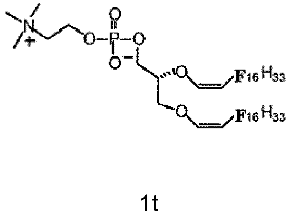
- Orthoester spaltbar durch Umlagerung

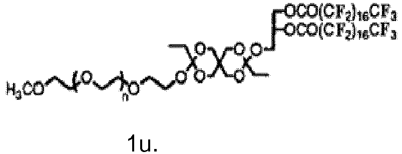

In einer Ausführungsform der vorliegenden Verbindung ist das mindestens eine Linkermolekül C ausgewählt aus der Gruppe enthaltend geradlinige oder vezweigte acyclische oder cyclische, polycyclische oder heterocyclische aliphatische Alkane, Alkene, Alkine oder aromatische Verbindungen.

Das verwendete Linkermolekül C wird bevorzugt verwendet, um in der vorliegenden Verbindung eine oder mehrere Bindestellen für weitere, zusätzliche Moleküle wie z.B. Markermoleküle G' und/oder Liganden F' oder andere Erkennungssequenzen zur Verfügung zu stellen.

Als Linkermolekül C werden hiermit Moleküle offenbart, die ausgewählt aus einer Gruppe enthaltend insbesondere Halogenalkane, Hydroxyl-, Ether-, Amino-, Sulhydryl-, Aldehyd-Keto-, Carboxyl-, Ester- und Säureamidgruppen, Gruppen mit Radikalen oder Ionen und Moleküle aus den Stoffgruppen der Carbonsäuren, Peroxycarbonsäuren, Thiocarbonsäuren, Sulfonsäuren, Sulfinsäuren, Sulfensäuren, Sulfoxiden, Carbonsäuresalzen, Sulfonsäuresalzen, Sulfinsäuresalzen, Sulfensäuresalzen, Carbonsäureanhydriden, Carbonsäureestern, Sulfonsäureestern, Carbonsäurehalogeniden, Sulfonsäurehalogeniden, Carbonsäureamiden, Sulfonsäureamiden, Carbonsäurehydraziden, Nitrilen, Aldehyden, Thioaldehyden, Ketonen, Thioketonen, Oximen, Alkoholen, Phenolen, Thiolen, Aminen, Iminen, Hydrazinen, Ethern, Estern, Thioethern, Thioestern, Halogenwasserstoffen, Nitroverbindungen, Nitrosoverbindungen, Azoverbindungen, Diazoverbindungen, Diazoniumsalzen, Isocyanaten, Cyanaten, Isocyaniden, Thiocyanaten, Isothiocyanaten, Hydroperoxiden, Peroxiden oder Verbindungen, die aufgrund ihrer Mehrfachbindung reaktiv sein können, oder funktionalisierte Perfluorkohlenwasserstoffe mit Jod-, Brom- oder Schwefelatomen, Carbamaten, Thioether- oder Disulfidgruppen, Glycerol, Succinylglycerol, Phosphatgruppen sowie funktonalisierte Perluorkohlenwasserstoffe mit anderen Gruppen oder Atomen, über die eine Verbindung zu Nukleosiden, Nukleotiden, Oligonukleotiden, Nukleinsäuren, modifizierte Nukleosiden, modifizierten Nukleotiden, modifizierten Oligonukleotiden, modifizierten Nukleinsäuren, Peptid-Nukleosiden, Peptid-Nukleotiden, Peptid-Oligonukleotiden, Peptid-Nukleinsäuren oder pharmazeutische Substanzen hergestellt werden kann.

Offenbarte Linkermoleküle C basieren auf Hydroxyl- oder Aminogruppen, Carboxylgruppen, Ester, Ether, Thioether, Thioester, Carbonsäureamide, Verbindungen mit Mehrfachbindungen, Carbamate, Disulfidbrücken und Hydrazide, Halogenalkane, Sulhydryl-, Aldehyd- Keto-, Carboxyl-, Ester- und Säureamidgruppen, Thiolen, Aminen, Iminen, Hydrazinen, oder Disulfidgruppen, Glycerol, Succinylglycerol, Orthoester, Phosphorsäurediester und Vinylether, wobei Ester- und Ethergruppen und Disulfidbrücken ganz besonders bevorzugt sind.

Wie oben beschrieben kann das Linkermolekül C zur Anbindung von weiteren Markern G' und/oder Liganden F' oder anderen Erkennungssequenzen dienen, die im wesentlichen die gleichen Gruppen wie der weiter unten definierte Ligand F und Marker G umfassen, sich von diesen allerdings in der Anordnung innerhalb der vorliegenden Verbindung unterscheiden. Geeignete Beispiele für den Marker G' sind hier Fluoreszenz-Farbstoffe wie Dil, DilC, DiO, Fluorescine, Rhodamine, Oxacine, Fuchsine, Pyronine, Acridine, Auramine, Pararosaniline, GFP, RFP, DAPI, oder Peroxidase-Farbstoffe wie ABTS.

Liganden und Erkennungssequenzen werden zum spezifischen Transfer in bestimmte Zelltypen benötigt, da sie an Rezeptoren auf der Zelloberfläche binden und dadurch einen spezifischen Zelleintritt ermöglichen. Liganden werden in der Regel über zugängliche Seiten- oder endständige Aminogruppen am Konstrukt gebunden. Bindungen über andere Gruppen sind allerdings möglich.

Als geeignete Liganden F' können vorliegend Transferrin, Folsäure, Galactose, Lactose, Mannose, Epidermal Growth Factor, RGD-Peptide, Biotin oder eine Kernlokalisationssequenz verwendet werden.

Spacermolekül D sind hiermit offenbart, die ausgewählt aus der Gruppe enthaltend geradlinige oder verzweigte Aliphate mit einer oder mehreren funktionellen Gruppen, wobei das Spacermolekül D auch als Linkermoleül C dienen kann.

Es werden hiermit Spacermolekül offenbart, die verwendet werden, um den verschiedenen Gruppen im Molekül mehr Raum zur Vermeidung von sterischen Behinderungen zu geben oder um die negative Ladung der Fluorbausteine auf andere Molekülbereiche abzuschwächen. Besonders bevorzugt ist das Spacermolekül D aus der Gruppe der Fettsäurealkohole, Fettsäurediole und Fettsäurepolyole ausgewählt.

In einer bevorzugten Ausführungsform der beschriebenen Verbindung werden als Molekül E Nukleobasen ausgewählt aus der Gruppe enthaltend Adenin, Guanin, Hypoxanthin, Xanthin, Cytosin, Uracil, Thymin, modifizierte Nukleobasen wie 5-Bromuracil, 5-Fluoruracil, Zidovudine, Azidothymidine, Stavudin, Zalcitabin, Diadenosin, Idoxuridin, Fluridin und Ribavirin, Azidothymidin, Zidovudin, 5-Methyluracil, 5-Methylcytosin, 5-Fluorcytosin, 5-Bromcytosin, 2-Aminopurin und deren "Spiegelmere" verwendet, wobei die Nukleobasen Adenin, Guanin, Cytosin, Uracil, Thymin besonders bevorzugt sind.

Bevorzugterweise werden in den beschriebenen Verbindungen als Molekül E Nukleoside ausgewählt aus der Gruppe enthaltend Adenosin, Guanosin, Cytidin, 5-Methyluridin, Uridin, Desoxyadenosin, Desoxyguanosin, Thymidin, Desoxyuridin, Desoxycytidin oder modifizierte Nukleoside wie 2-Thiocytidin, N4-Acetylcytidin, 2'-O-Methylcytidin, 3-Methylcytidin, 5-Methylcytidin, 2-Thiouridin, 4-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxyuridin, 2'-O-Methyluridin, Ribothymidin, 1-Methyladenosin, 2-Methyladenosin, N6-Methyladenosin, N6-Isopentenyladenosin, 2'-O-Methyladenosin, Inosin, 1-Methylinosin, 1-Methylguanosin, N2-2-Methylguanosin, N2-2,2-Dimethylguanosin, 7+-Methylguanosin, 2'-O-Methylguanosin, Queuosin , β-D-Galactosyl-queuosin, β-D-Mannosyl-queuosin, Archaeosin , 2'-O-Ribosyladenosinphosphat , N6-Threonylcarbamoyladenosin, Lysidin, Nicotinsäure, Riboflavin und Pantothensäure, NADPH, NADH, FAD, Coenzym A, und Succinyl-Coenzym A, Puromycin, Aciclovir, Ganciclovir und deren "Spiegelmere" verwendet, wobei die Nukleoside Adenosin, Guanosin, Uridin, Cytidin, Desoxyadenosin, Desoxyguanosin, Desoxycytidin, Desoxythymidin besonders bevorzugt sind.

In einer weiteren beschriebenen bevorzugten Ausführungsform werden als Molekül E Nukleotide ausgewählt aus der Gruppe enthaltend AMP, GMP, m5UMP, UMP, CMP, dAMP, dGMP, dTMP, dUMP, dCMP, cAMP, cGMP, c-di-GMP, cADPR, ADP, GDP, m5UDP, UDP, CDP, dADP, dGDP, dTDP, dUDP, dCTP, ATP, GTP, m5UTP, UTP, CTP, dATP, dGTP, dTTP, dUTP, dCTP oder modifizierte Nukleotide, die aus oben beschriebenen Bausteinen hervorgehen, Nukleotide mit Modifikationen am Zucker-Phosphatgerüst, zwitterionische Oligonukleotide sowie Nukleotide, bei denen das Phoshphat ersetzt wurde durch Methylphosphonat- oder Dimethylsulfongruppe, wobei AMP, GMP, m5UMP, UMP, CMP, dAMP, dGMP, dTMP, dUMP, dCMP besonders bevorzugt sind.

Erfindungsgemäß ist es bevorzugt als Molekül E Desoxyribonukleinsäuren oder Ribonukleinsäuren zu verwenden. Ebenfalls beschrieben ist die Verwendung von modifizierten Nukleinsäuren, wie z.B. Nucleosidphosphorothioate, zwitterionische Nukleinsäuren, Nukleinsäuren bei denen das Phoshphat getauscht wurde gegen eine Methylphosphonat- oder Dimethylsulfongruppe, verbrückte Nukleinsäuren (locked nucleic acids), "Spiegelmere", Nukleinsäuren, bei denen das Ribose-Phosphodiester-Rückgrates gegen verschiedene polymere Konstrukte getauscht wurde wie z.B. ein auf Hexitol basierender Rückgratstrang oder ein auf Glycerineinheiten basierendes Nukleinsäure-Analogon), Morpholino-Oligonukleotide, Phosphorthioat-Desoxyribonukleinsäure, Cyclohexen-Nukleinsäuren, N3'-P5'-Phosphoramidate, Tricyclo-Desoxyribonukleinsäuren, Morpholino-Phosphoramidate-Nukleinsäuren, Threose-Nukleinsäuren, wobei Nucleosidphosphorothioate, Phosphorthioat-Desoxyribonukleinsäure besonders bevorzugt sind.

Werden bevorzugterweise als Molekül E einzelsträngige oder doppelsträngige Oligonukleotide und Nukleinsäuren verwendet, so können diese eine Länge von 2 Basenpaaren bis größer als 1.000.000 Bp aufweisen, wobei folgende Längenbereiche jeweils bevorzugt sind: 10 bis 50 bp, 15 bis 25 bp, 25 bis 200 bp, 25 bis 100 bp, 200 bis 300 bp, 200 bis 500 bp, 500 bis 1500 bp, 800 bis 1300 bp, 1500 bis 20.000 bp, 1500 bis 5000 bp, 3000 bis 8000 bp, 20.000 bis 1.000.000 bp oder 20.000 bis 50.000, wobei ganz besonders bevorzugt Oligonukleotide mit einer Länge zwischen 10 bis 50 bp, 200 bis 500 bp und 500 bis 1500 bp bevorzugt sind.

Es wird hiermit offenbart, dass das Molekül E mit funktionellen Gruppen ausgewählt aus der Gruppe enthaltend Hydrazide, Halogenalkane, Hydroxyl-, Ether-, Amino-, Sulhydryl-, Aldehyd- Keto-, Carboxyl-, Ester- und Säureamidgruppen, Gruppen mit Radikalen oder Ionen und Moleküle aus den Stoffgruppen der Carbonsäuren, Peroxycarbonsäuren, Thiocarbonsäuren, Sulfonsäuren, Sulfinsäuren, Sulfensäuren, Sulfoxiden, Carbonsäuresalzen, Sulfonsäuresalzen, Sulfinsäuresalzen, Sulfensäuresalzen, Carbonsäureanhydriden, Carbonsäureestern, Sulfonsäureestern, Carbonsäurehalogeniden, Sulfonsäurehalogeniden, Carbonsäureamiden, Sulfonsäureamiden, Carbonsäurehydraziden, Nitrilen, Aldehyden, Thioaldehyden, Ketonen, Thioketonen, Oximen, Alkoholen, Phenolen, Thiolen, Aminen, Iminen, Hydrazinen, Ethern, Estern, Thioethern, Thioestern, Halogenwasserstoffen, Nitroverbindungen, Nitrosoverbindungen, Azoverbindungen, Diazoverbindungen, Diazoniumsalzen, Isocyanaten, Cyanaten, Isocyaniden, Thiocyanaten, Isothiocyanaten, Hydroperoxiden, Peroxiden oder Gruppen, die aufgrund ihrer Mehrfachbindung reaktiv sein können oder funktionalisierte Perfluorkohlenwasserstoffe mit Jod-, Brom- oder Schwefelatomen, Carbamaten, Thioether- oder Disulfidgruppen, Glycerol, Succinylglycerol, Phosphatgruppen oder andere funktionelle Gruppen, mit denen eine Bindung an einen funktionalisierten Perfluorkohlenwasserstoff gemacht werden kann, versehen ist.

Die funktionellen Gruppen sind als weitere Substituenten am Nukleotid zu verstehen. Beispielsweise kann an der 2'H-Position der Desoxyribose eines Nukleosids oder Nukleotids eine NH₂-Gruppe eingeführt, um diese Stelle, die ursprünglich nicht zur Perfluorierung nutzbar war, für eine Perfluorierung vorzubereiten. Auch kann z.B. an der 2'OH-Position der Ribose eine SH-Gruppe eingeführt, um später eine Disulfidbrücke zu erzeugen.

Bevorzugte Substituenten bzw. funktionelle Gruppen am Molekül E am sind -OH, -NH₂ und - SH Gruppen, Hydrazide, Halogenalkane, Sulhydryl-, Aldehyd- Keto-, Carboxyl-, Ester- und Säureamidgruppen, Ether, Thioester, Thioether,

Der in der beschriebenen Verbindung vorhandene Ligand F ist bevorzugterweise ausgewählt aus einer Gruppe enthaltend Transferrin, Folsäure, Galactose, Lactose, Mannose, Epidermal Growth Factor, RGD-Peptide und Biotin.

Das in der beschriebenen Verbindung vorhandene Markermolekül G ist bevorzugterweise ausgewählt aus einer Gruppe enthaltend Fluoreszenz-Farbstoffe wie Dil, DilC, DiO, Fluorescine, Rhodamine, Oxacine, Fuchsine, Pyronine, Acridine, Auramine, Pararosaniline, GFP, RFP und DAPI.

Die vorliegende Verbindung ist insbesondere zum nicht-viralen Transfer von mindestens einem Molekül E in mindestens eine Zelle eines eukaryotischen Organismus, insbesondere von Tieren oder Menschen, geeignet und einsetzbar.

Besonders bevorzugt wird die vorliegende Verbindung in Form einer pharmazeutischen Zusammensetzung zusammen mit mindestens einer oberflächenaktiven Substanz verwendet, wobei geeignete oberflächenaktive Substanzen z.B. Poloxamere, Lecithine oder andere zellverträgliche Tenside sind.

Die pharmazeutische Zusammensetzung kann in Form einer Emulsion, Dispersion, Suspension oder Lösung insbesondere mit einer mittleren Partikelgröße zwischen 2 nm und 200 µm, bevorzugt zwischen 20 nm und 400 nm, insbesondere bevorzugt bei 50 nm vorliegen. Die Partikelgröße kann abhängig von der Verwendung variieren. Durch geeignete Methoden z.B. Sonicator, Zerstäubung oder Lösungsmittel-Methode kann die gewünschte Partikelgröße eingestellt werden, die günstig für die Aufnahme in die Zelle ist. Auch ist die vorliegende pharmazeutische Zusammensetzung zum nicht-viralen Transfer von mindestens einem Molekül E in mindestens eine Zelle eines eukaryotischen Organismus, insbesondere von Tieren oder Menschen geeignet und einsetzbar.

Die vorliegenden Verbindungen können in verschiedener Weise hergestellt und modifiziert werden. Insbesondere kann die Verknüpfung des Moleküls E mit den perfluorierten Verbindungen wie Perfluorkohlenwasserstoffe (PFC) an verschiedenen Positionen des Moleküls E erfolgen, die hierfür zugänglich sind.

Als Perfluorierungsstelle bzw. - position im Sinne der vorliegenden Anmeldung ist dabei insbesondere die Stelle im Molekül E zu verstehen, an welcher die Verknüpfung des Moleküls E mit dem perfluorierten Molekül A oder A' bevorzugt über eine Sollbruchstelle B bei optionaler Verwendung eines Linkermoleküls C und/oder Spacermoleküls D erfolgt.

Im Folgenden werden bevorzugte Perfluorierungsposition für verschiedene Moleküle E angeführt: a) Perfluorierung von Nukleobasen, Nukleosiden, Nukleotiden, b) Perfluorierung von Peptid-Nukleinsäure-Monomeren und Oligomeren, Peptid-Nukleinsäuren, c) Perfluorierung von Oligonukelotiden.

In einer ersten Variante kann die Perfluorierung von Nukleobasen als Molekül E an allen zugänglichen Stellen im Molekül erfolgen, wobei bevorzugte Perfluorierungsstellen NH₂- und NH-Gruppen sind. Eine Limitierung erfolgt lediglich bei einem Umbau zum Nukleosid an der betreffenden NH-Gruppe (Pfeil), siehe Schema 2.

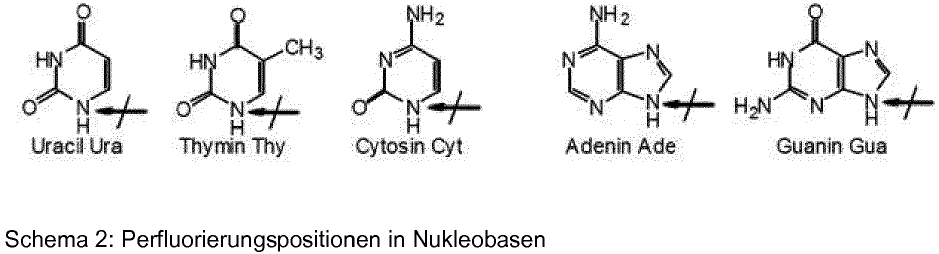

In einer zweiten Variante ist eine Perfluorierung auch an Perfluorierungsstellen im Zuckermolekül von Nukleosiden möglich, wobei die Perfluorierungsstelle die 2'-, Position- der Ribose ist. Die entsprechenden Positionen der Ribose können auch modifiziert in Form von - NH₂ und/oder -SH vorliegen, wobei die bevorzugten Perfluorierungsstellen entsprechend 2'-NH₂, 2'-SH usw. sind; siehe Schema 3.

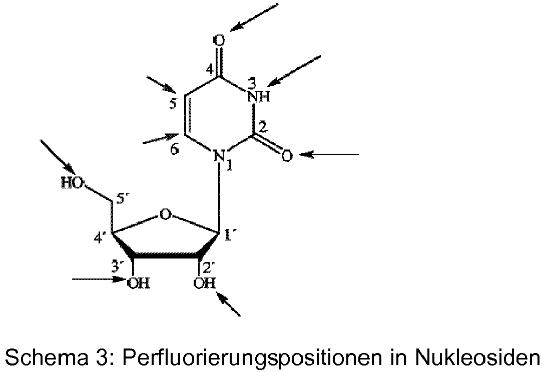

Für die Perfluorierung von Nukleotiden ergeben sich in einer dritten Variante neben den Perfluorierungspositionen an der Ribose in 2' Position zusätzliche Perfluorierungsstellen an der Phospatgruppe, wie z.B. an der freien OH-Gruppe oder an mindestens einem Sauerstoffatom; siehe Schema 4.

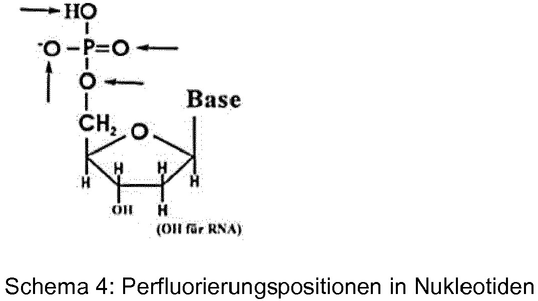

Es können sich allerdings alternativ auch Perfluorierungsstellen an modifizierten Zucker-Phosphat-Gerüsten mit Heteroatomen wie beispielsweise S oder N oder andere ergeben, wie z.B. in Schema 5 gezeigt.

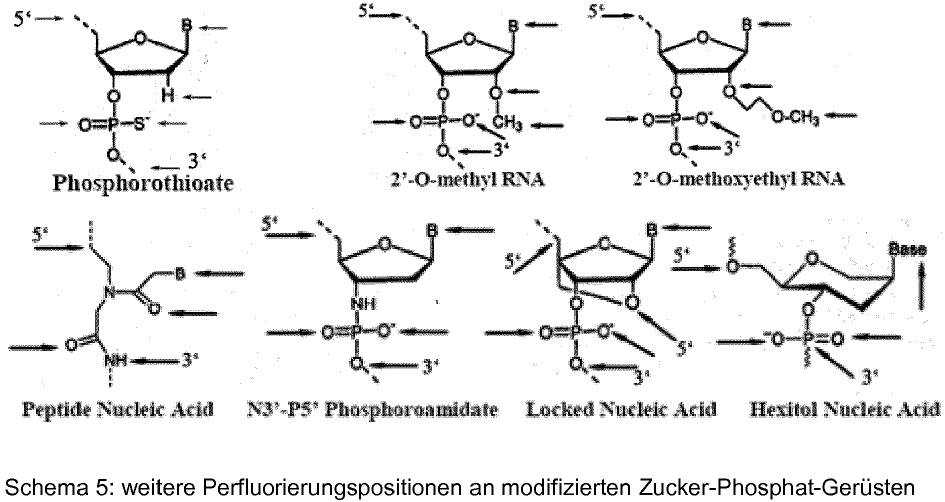

Hiermit wird in einer vierten Variante - die nicht Gegenstand der Erfindung ist - offenbart, dass die Perfluorierung von Peptid-Nukleinsäure-Monomeren, Peptid-Nukleinsäure-Oligomeren und Peptid-Nukleinsäuren erfolgen kann. Diese sind Analoga zu den Nukleinsäuren. Das Zucker-Phosphat-Rückgrat ist ersetzt durch ein Pseudopeptid, beispielsweise durch Aminoethylglycin-Einheiten, die über neutrale Amid-Bindungen miteinander verbunden sind. Sie sind sehr stabil, da sie sowohl von Nukleasen als auch von Proteasen nicht abgebaut werden können. Sie hybridisieren stringenter mit komplementären DNA- und RNA-Sequenzen als ursprüngliche Oligomere, zusätzliche Perfluorierungsstellen existieren an NH₂- und Carboxygruppen und am O-Atom sowie an funktionellen Gruppen modifizierter Peptidgerüste; siehe Schema 6.

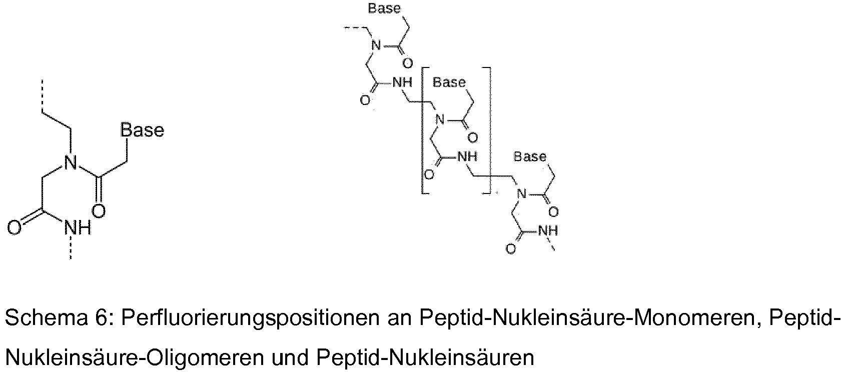

In einer fünften Variante kann die Perfluorierung von Nukleinsäure-Oligomeren bzw. Oligonukelotiden und von Nukleinsäure-Makromolekülen erfolgen, wobei hier verschiedene Ansätze möglich sind.

In einem ersten Ansatz erfolgt der direkte Einbau von perfluorierten Nukleotiden in die gewünschte RNA- oder DNA-Sequenz, z.B. mittels Festphasensynthese oder mittels PCR, wobei bevorzugt perfluorierte Nukleotide verwendet werden, die an 2'-Stelle (d.h. in der 2'-Position der Ribose) perfluoriert sind. Das liegt darin begründet, dass einerseits Perfluorierungen an dieser Stelle nicht zum Kettenabbruch bei einer Polymerisation bzw. Synthese führen und andererseits die Wechselwirkungen zwischen Basenpaaren nicht gestört werden; siehe die zwei oberen Beispiel des Schemas 7.

Während bei RNA-Molekülen die 2'OH-Position einfach zu perfluorieren ist, muss bei DNA-Molekülen in der Regel die 2'H-Position zunächst funktionalisiert werden, beispielsweise durch perfluorierbare Gruppen wie NH₂ statt H oder 2'-Amino-2'-desoxyuridin.

In einem zweiten Ansatz erfolgt bevorzugt ein Einbau von RNA-Nukleotiden in die DNA-Sequenz bzw. Einbau perfluorierter DNA-Nukleotide an den 5' oder 3'-Enden des Oligonukleotids oder des DNA-Makromoleküls; siehe drittes Beispiel in Schema 7. In diesem Ansatz werden demnach an die Enden des fertigen Oligonukleotids perfluorierte Verbindungen wie perfluorierte Nukelotide bevorzugt mittels chemischer Synthese angefügt. Die verwendeten Nukleotide können an jeder möglichen Position wie oben beschreiben perfluoriert sein, wobei 2' -perfluorierte Nukleotide bevorzugt sind.

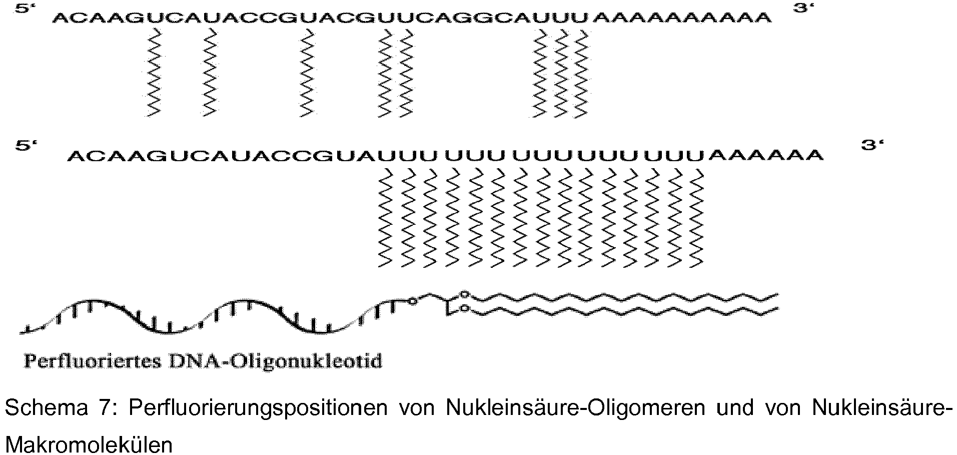

Die Verbindungen können in unterschiedlichen Konstrukten mit unterschiedlichem Aufbau vorliegen, d.h. die Verbindungen können in Abhängigkeit der Präsenz der Moleküle C, D, F und G folgende weitere Grundstrukturen

- A-B-E-F-G, (III)

- A-B-D-B-E-F-G, (IV)

,

- A-B-C-B-E-F-G, (V)

,

- A-B-E, (VI)

,

- A-B-D-B-E, (VII)

,

- A-B-C-B-E (VIII)

,

- A-B-E-F, (IX)

- A-B-D-B-E-F, (X)

,

- A-B-C-B-E-F, (XI)

,

- A-B-E-G, (XII)

- A-B-D-B-E-G, (XIII)

,

- A-B-C-B-E-G, (XIV)

,

- A-B-E-A', (XV)

,

- A-B-D-B-E, (XVI)

,

- A-B-C(F')-B-E (XVII)

,

- A-B-C(G')-B-E, (XVIII)

- A-B-C(F', G')-B-E, (XIX)

,

- A-B-C(F')-E, (XX)

,

- A-B-C(G')-E, (XXI)

- A-B-C(F', G')-E, (XXII)

aufweisen. Erfindungsgemäß sind die Grundstrukturen (XIX) und (XXII). Folgende Verbindungen werden hiermit offenbart:

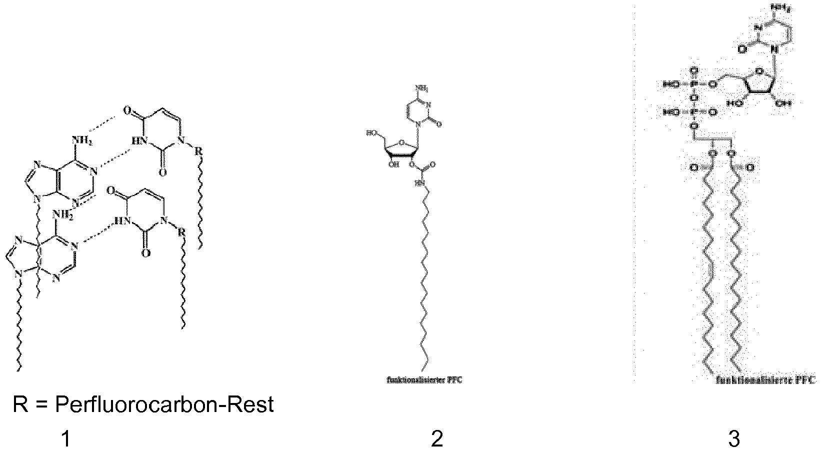

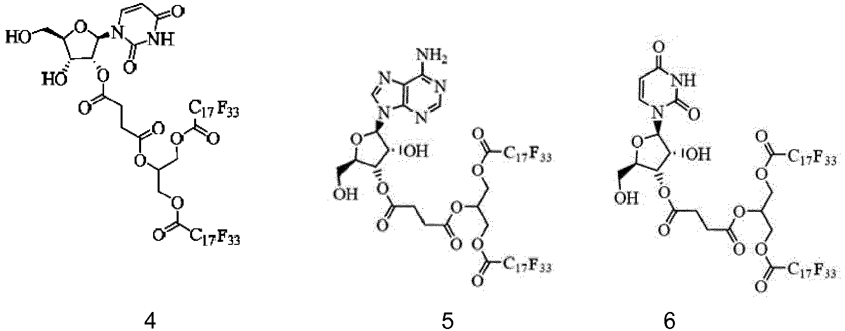

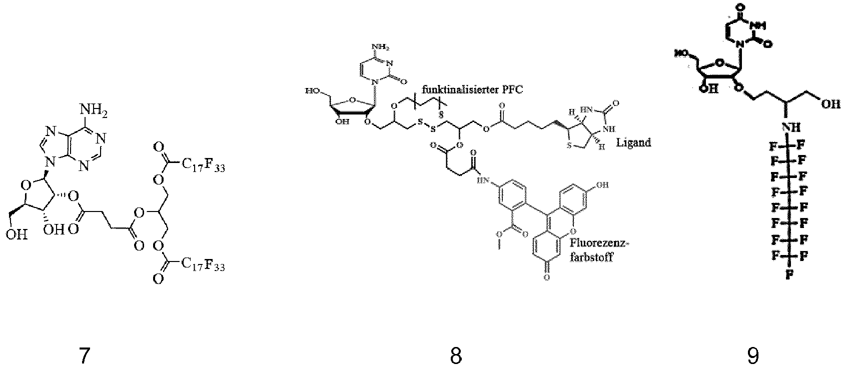

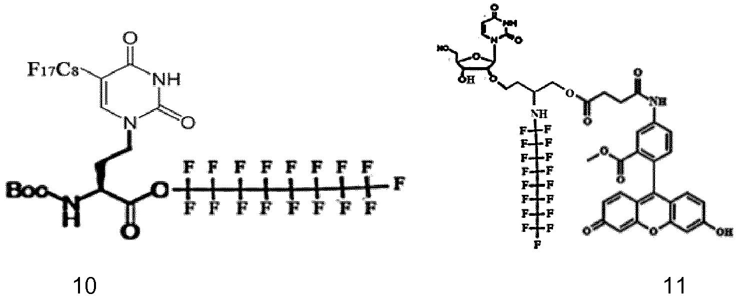

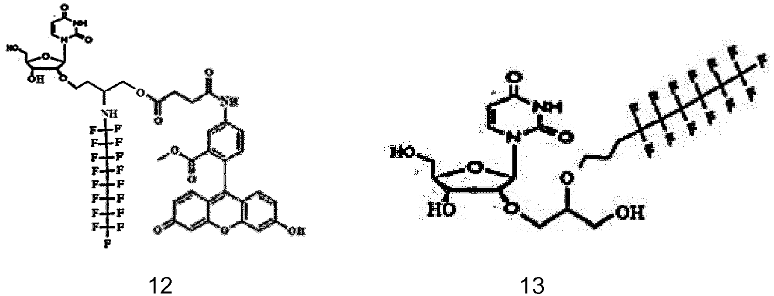

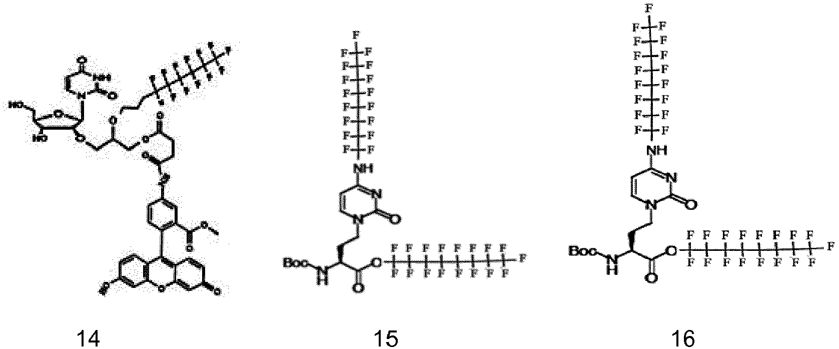

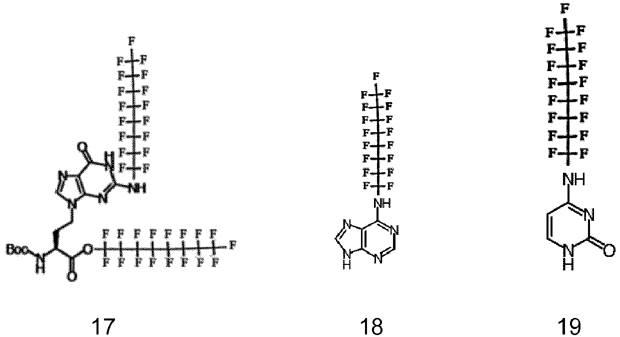

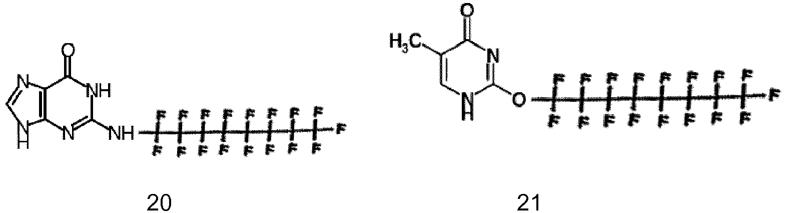

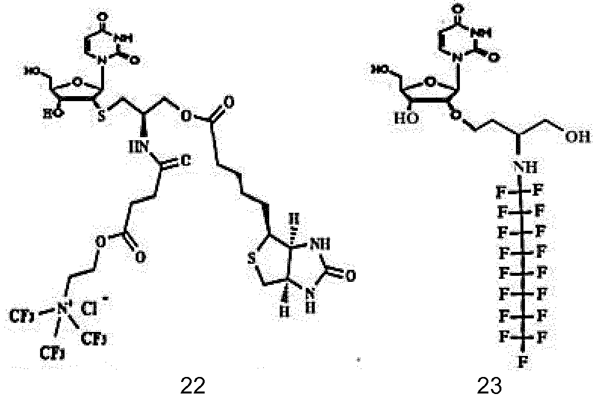

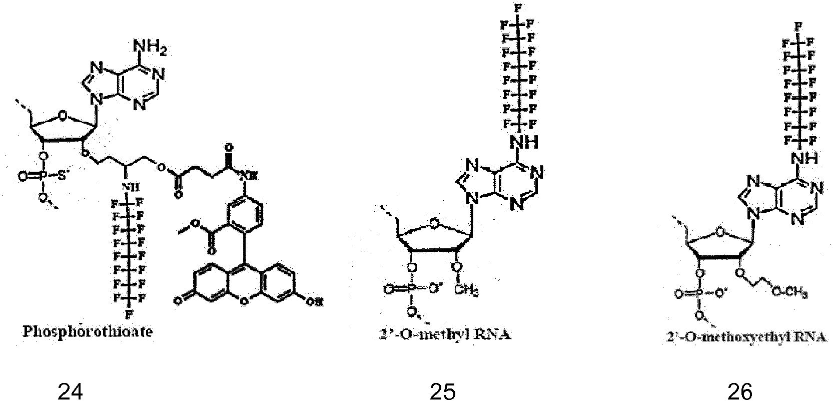

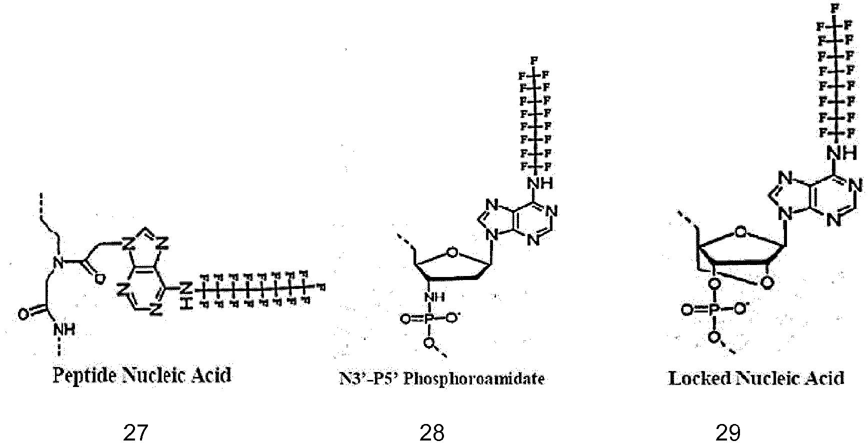

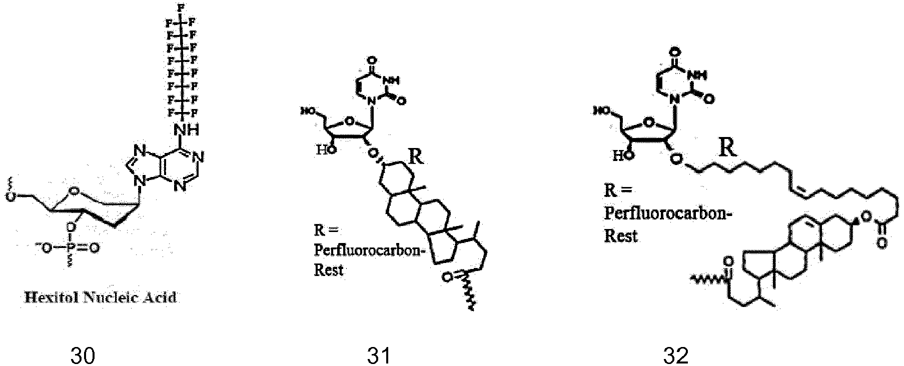

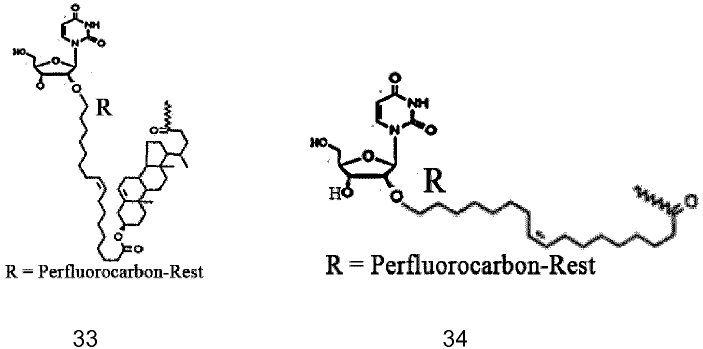

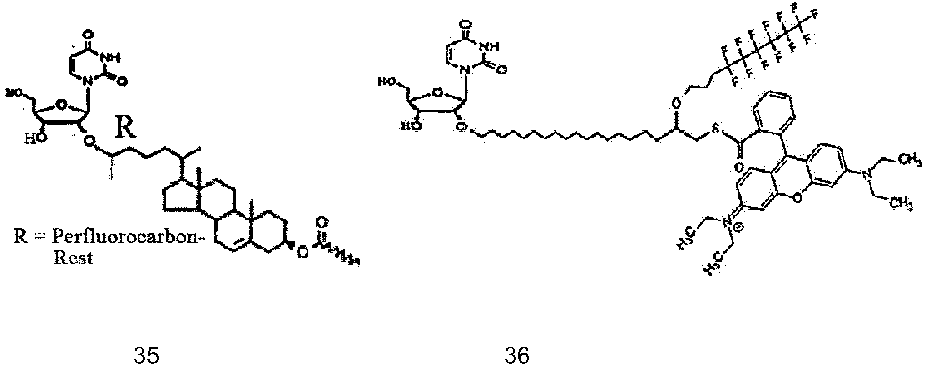

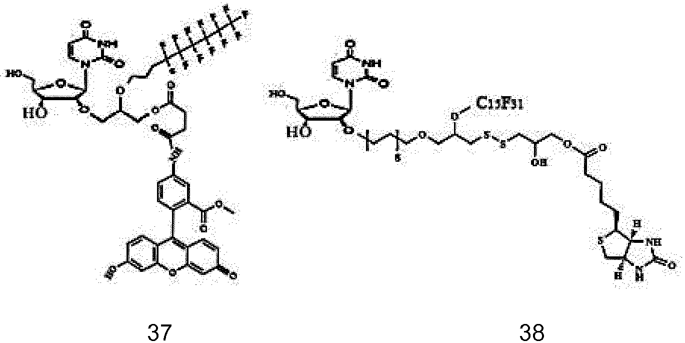

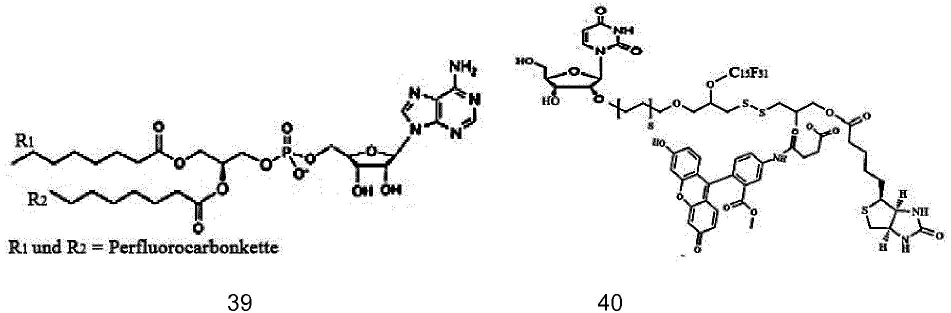

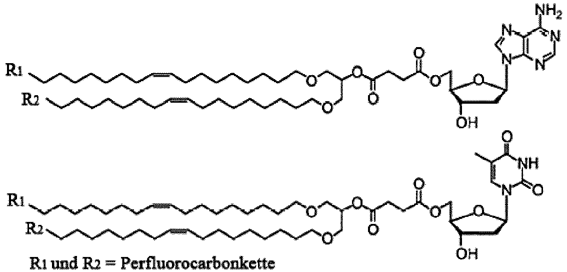

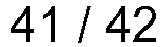

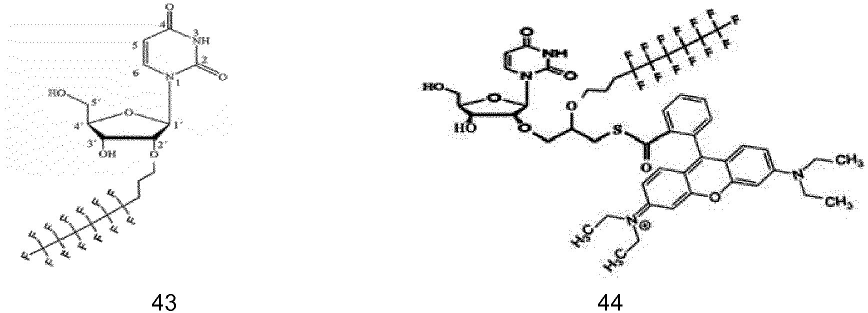

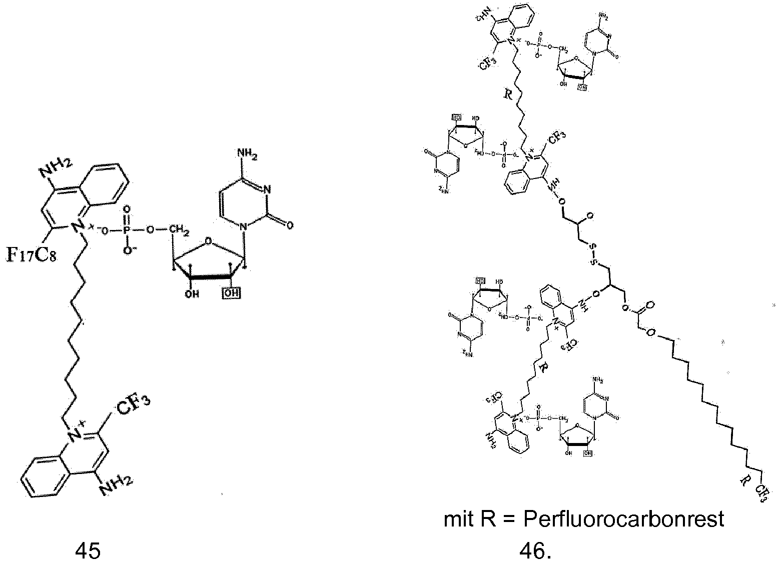

Die Verbindungen der vorliegenden Verfahren werden mittels bekannter Syntheseverfahren hergestellt, die eine sukzessive Verknüpfung der einzlnen Molekülbausteine z.B. mittels Addition, Substitution, Kondensation, Veretherung, Veresterung ermöglichen. Derartige Synthesewege sing einen Synthesechemiker als Fachmann bekannt.

Zum besseren Verständnis wirdg vorliegende Erfindung und nicht erfindungsgemäße Ausführungsformen im Folgenden anhand von Ausführungsbeispielen unter Bezugnahme auf die Figuren näher erläutert. Der Gegenstand der Erfindung wird durch die Ansprüche definiert und beschränkt sich nicht auf die erfindungsgemäßen Beispiele. Es zeigen:
- Figur 1: mikroskopische Aufnahmen von mit beschriebenen Verbindungen transfizierten Zellen, und
- Figur 2: FACS Analyse von mit beschriebenen Verbindungen transfizierten Zellen.

### Ausführungsbeispiel 1: Synthese von perfluorierten Nukleobasen

Die Perfluorierung von Nukleobasen erfolgt durch eine Williamson'schen Ethersynthese. Relativ leicht zugänglich sind hier die NH₂-Gruppen. Thymin besitzt keine NH₂-Gruppe, kann aber in 6 tautomeren Strukturen auftreten, wovon 4 Strukturen eine perfluorierbare OH-Gruppe aufweisen.

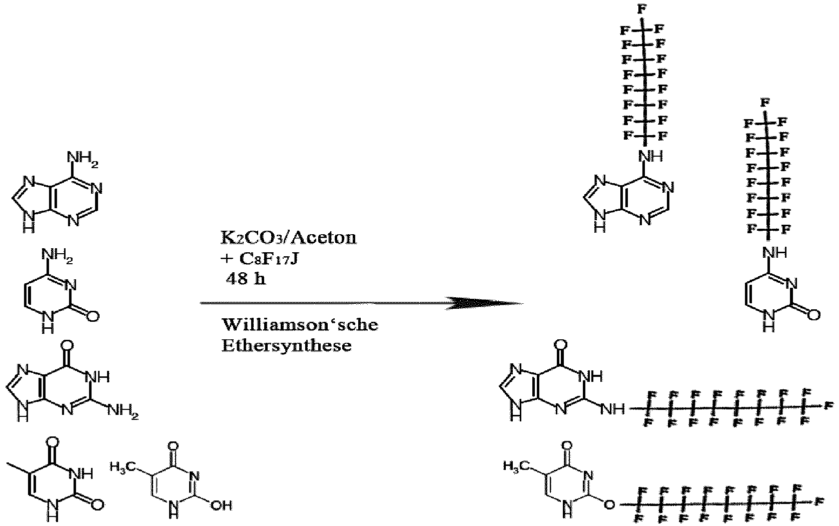

### Ausführungsbeispiel 2: Synthese von 2'-perfluorierten Nukleosiden anhand von 2'-perfluoriertem Uridin

Uridin ist ein wichtiger Bestandteil von RNA. Dabei erfolgt der Einbau in die RNA-Kette über die OH-Gruppen der 3'und 5'-Position im Zucker. Die 2'-Position von Uridin ist damit für die Einführung von Substituenten besonders geeignet ohne das die Bindungsstellen der Nucleobase beeinträchtigt werden. Verschiedene 2'-substituierte Uridine sind bekannt, bei denen die Verknüpfung über 2'-Ether bzw. Ester, 2'-Thioether bzw. Ester, 2'-Säureamide oder 2'-Carbamate oder auch über 2-C' erfolgt, siehe Schema 8.

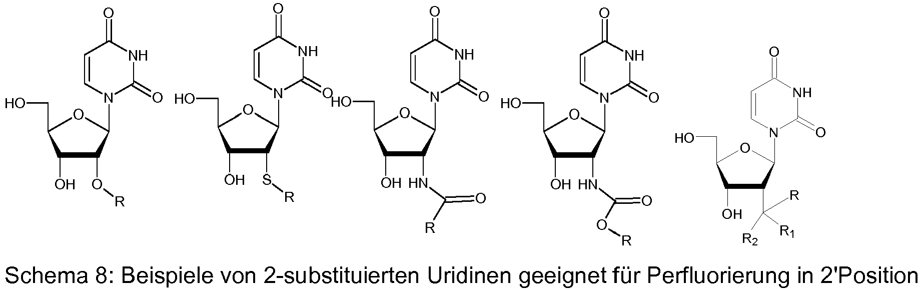

Für die Synthese von Uridinen mit Perfluoralkylen in der 2'-Position wurden neue Synthesen erarbeitet. So erfolgt die direkte Anbindung an die 2'-OH-Gruppe über eine Ether- und eine Esterfunktion, siehe Schema 9.

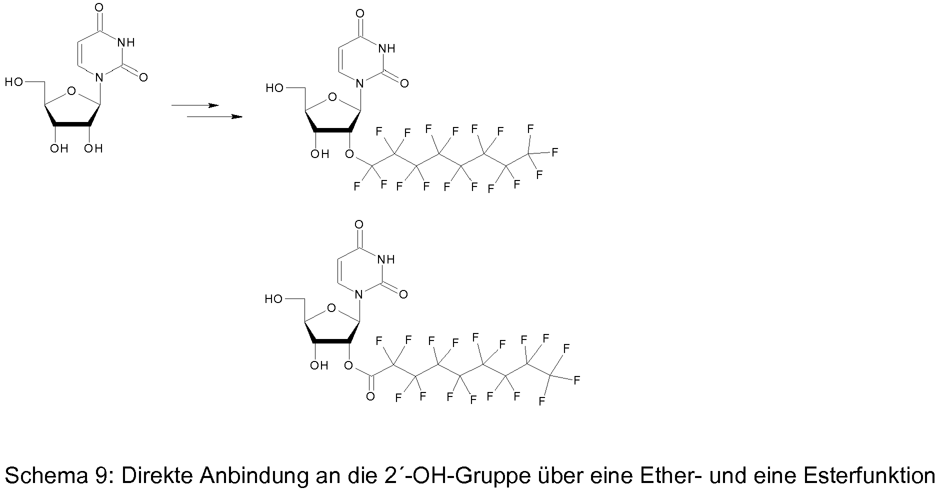

Die Anbindung an die 2'-OH-Gruppe ist auch über eine Ether- oder eine Esterfunktion unter Verwendung eines kurzen Spacers möglich, siehe Schema 10.

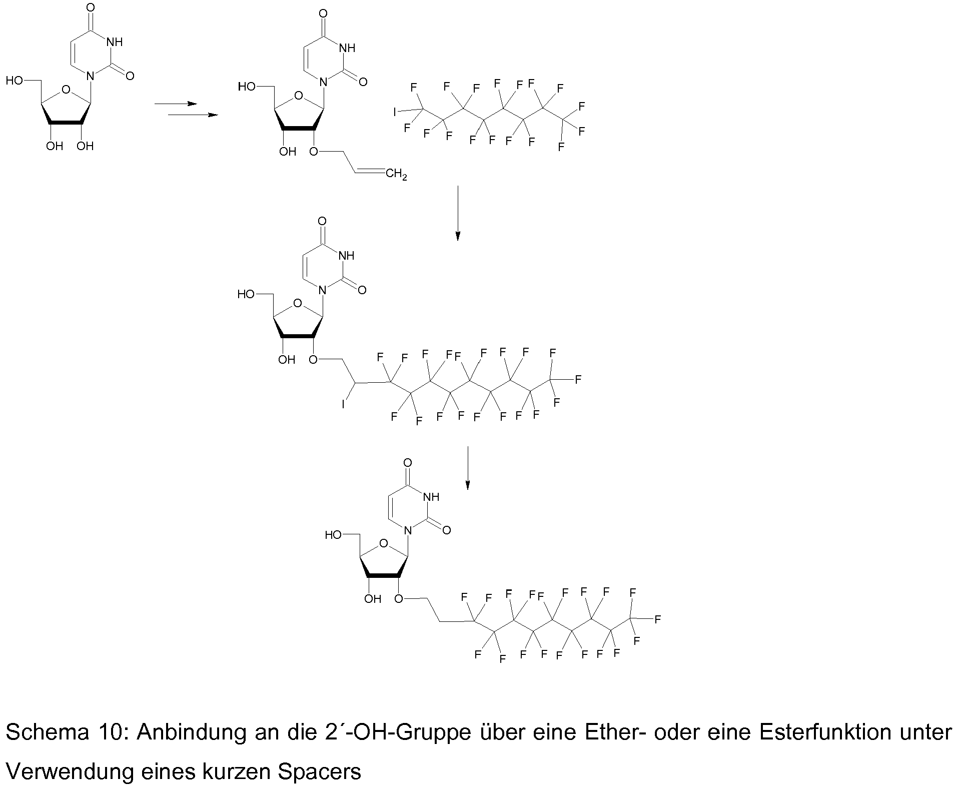

Eine weitere Möglichkeit besteht in der Anknüpfung des perfluorierten Alkylrestes über einen Spacer und eine Sollbruchstelle, siehe Schema 11.

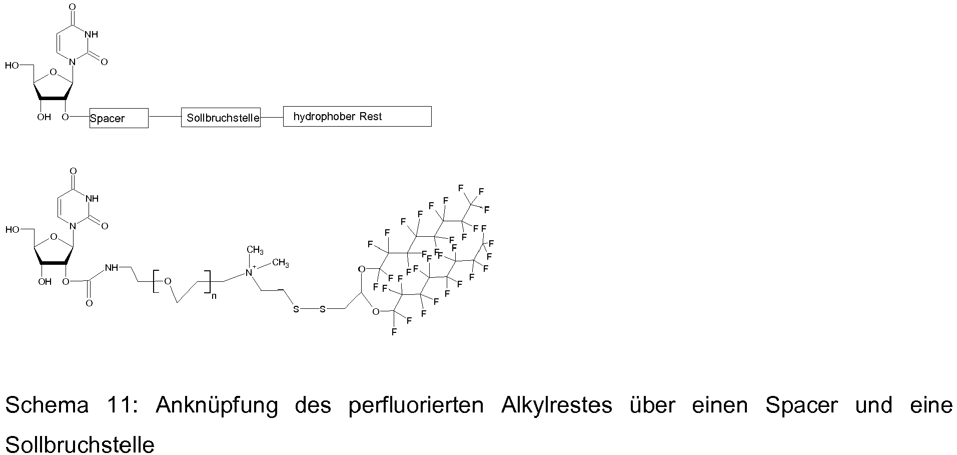

Für alle in den Schemen 9-11 formulierten Reaktionen müssen die 3'und 5'-OH-Gruppen geschützt werden. Dafür eignen sich Silylschutzgruppen. Dabei ist allerdings zu beachten, dass bei der Entschützung in einigen Fällen ein Gleichgewicht zwischen dem 2'und 3'-substituierten Uridin bilden kann (Acylmigration). Deshalb erfolgte die Anbindung des hydrophoben Restes an die 2'-Position des Uridins über eine Etherfunktion oder über eine Aminofunktion (2'-Amino-2'-desoxyuridin).

### Perfluorierung über eine 2'-Etherfunktion:

Die anschließende Einführung der perfluorierten hydrophoben Reste erfolgt über die 2'-OH-Gruppe über eine Etherfunktion. Dafür wurden im ersten Schritt die OH- Gruppen in 3'und 5' mit Tetraisopropyl-dichor-disilan geschützt und anschließend die OH-Gruppe in 2'-Position mit 1-lod-perfluoroktan bzw. 1-lod-perfluor-undekan verethert und im nächsten Schritt entschützt. Je nach Reaktionsverlauf wurden die Zwischen- und Endprodukte der Reaktionen über eine präparative Chromatographie gereinigt. Die Reaktionen wurden unter Ausschluss von Feuchtigkeit und unter Schutzgas (Argon) durchgeführt. Die verwendeten Lösungsmittel mussten ebenso vor ihrer Verwendung getrocknet werden.

Im Detail wird von einem funktionalisierten Perfluorkohlenwasserstoff (C8F17Br oder C8F17J) und einem Uridin ausgegangen, wobei zunächst am Uridin die 3'OH- und 5'-OH-Gruppen mit Schutzgruppen (3', 5'- Dietherbutylsiloxan) versehen werden. Die Reaktion mit dem funktionalisiertem Perfluorkohlenwasserstoff findet an der 2'OH-Gruppe des Uridins statt. Dazu wird in einer Williamson'schen Ethersynthese C8F17Br (oder C8F17J) an die 2'OH-Gruppe des Uridins gebunden (unter K2CO3/Aceton), wobei Uridin-2'0-C8F17 (mit 3', 5'- Dietherbutylsiloxan) entsteht. Die Reaktion mit dem funktionalisiertem Perfluorkohlenwasserstoff findet an der 2'OH-Gruppe des Uridins statt. Dazu wird eine Reaktion nach Monokanen et al. 1991 und Monokanen et al. 1993 durchgeführt, wobei Uridin-2'0-C8F17 (mit 3', 5'- Dietherbutylsiloxan) entsteht. Abschließend erfolgt die Abspaltung der Schutzgruppen; siehe Schema 12.

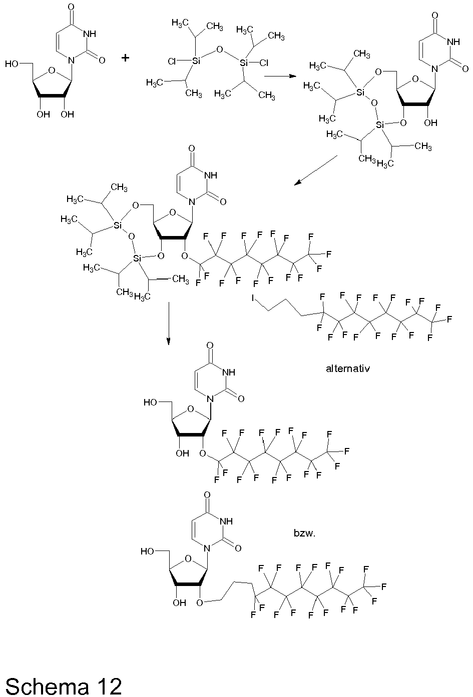

### Ausführungsbeispiel 3: Perfluorierung über eine 2'-Aminofunktion von 2'-Amino-2'-desoxyuridin

Die zweite Syntheseschiene geht von 2'-Amino-2'-desoxyuridin aus, das kommerziell verfügbar ist. Dieses wurde mit Perfluorcarbonsäuren zum Säureamid umgesetzt. Auch hier wurde unter Schutzgas und Ausschluß von Feuchtigkeit gearbeitet. Das Endprodukt wurde über präparative Säulenchromatographie gereinigt. Die primäre OH-Gruppe in 5'-Position des Uridins wurde mit DMT- geschützt. Für die analogen Reaktionen an anderen Positionen der Nukleinsäurekomponenten können adäquate Schutzgruppen zugefügt bzw. Weggelassen werden; siehe Schema 13.

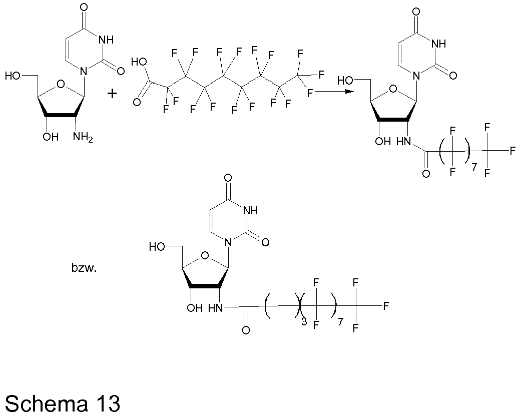

### Ausführungsbeispiel 4: Synthese eines perfluorierten Nukleosids mit Erkennungssequenz in Form von Biotin

Ausgangspunkt der Synthese war 2'modifiziertes Uridin-Nukleosid mit Aminoalkohol. Der Aminoalkohol wurde an seiner Aminofunktion mit mit C₈H₁₇OH perfluoriert. Durch die Verwendung von 1-Ethyl-3-(3-dimethylaminopropyl)carbodiimid (EDC), Hydroxybenzotriazol (HOBt) und Diisopropyl-ethylamin (DIPEA) konnte die Amidbildung chemoselektiv erreicht werden, ohne dass es zu einem Angriff an der alkoholischen Hydroxylgruppe kam. Die verbleibende primäre Hydroxylfunktion wurde anschließend mit Biotin verestert.

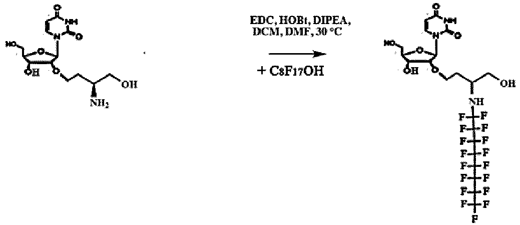

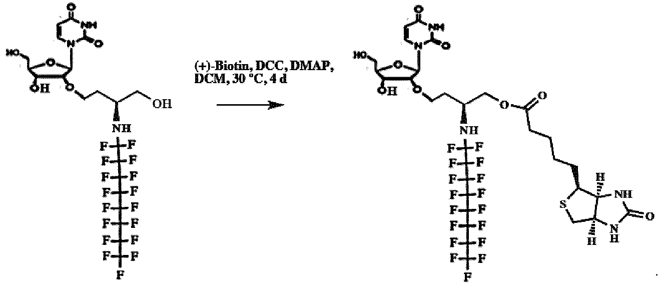

### Ausführungsbeispiel 5: Synthese eines perfluorierten Nukleosids mit Fluoreszenzfarbstoff

Es wurde ein Fluoresceinbaustein mit einer reaktiven Aminofunktion synthetisiert, so dass über eine Amidbindung die Ankopplung erfolgen konnte. Es wurde aus 4-Nitrophthalsäure und Resorcin das 5-Nitrofluorescein hergestellt. Bei der Kondensationsreaktion entstand ein Gemisch eines 6- und 5-Nitrofluorescein, das 6- Nitrofluorescein wurde durch fraktionierte Kristallisation abgetrennt. In der sich anschließenden Synthese wurde die Nitrofunktion zur Aminofunktion reduziert. Die Carboxylgruppe in der Verbindung wurde in einen Methylester umgewandelt. Die entstandene Verbindung wurde mit Succinsäureanhydrid acyliert und es entstand das entsprechende Amid.

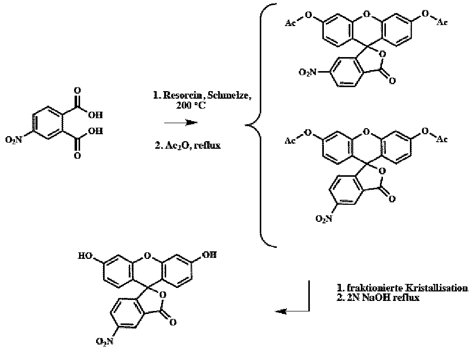

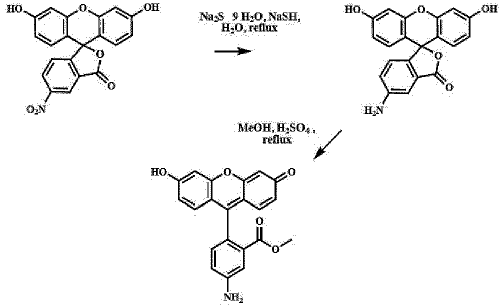

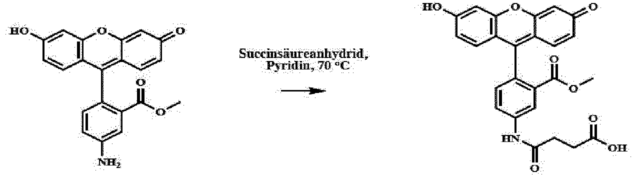

Die freie Carboxylfunktion wurde mit einer Hydroxylfunktion des perfluorierten Nukleotids verestert. 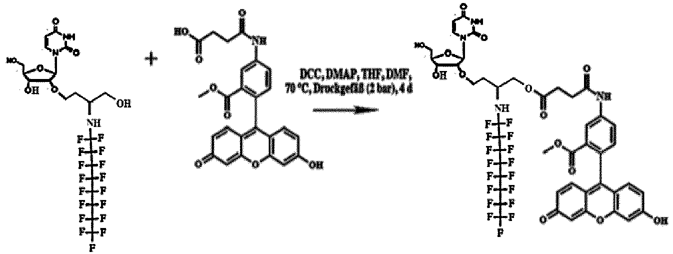 bzw.

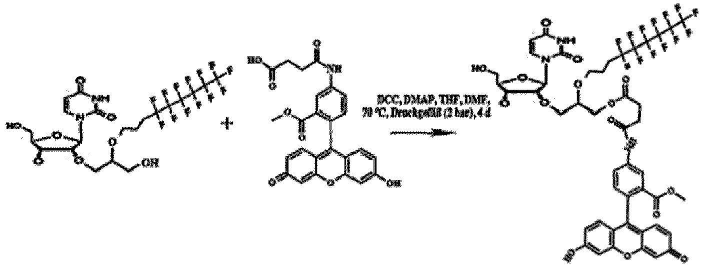

### Ausführungsbeispiel 6: Synthese eines perfluorierten Nukleosids mit Erkennungssequenz und Fluoreszenzfarbstoff

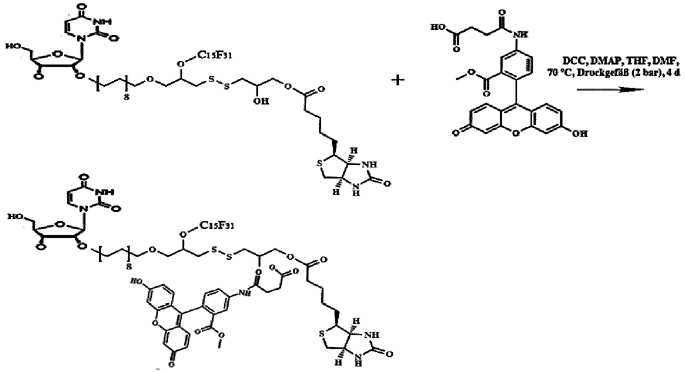

### Ausführungsbeispiel 7: Synthese von perfluorierten Oligonukleotiden und Nukleinsäuren

In einem ausgeheizten 50 ml Schlenkkolben werden unter Argonatmosphäre 0.90 mmol 2'-perfluoriertes Uridin in 20 ml trockenem Dichlormethan gelöst. Nachdem man der Lösung 1.00 mmol CyTIPP und 2.30 ml einer 0.45 M Tetrazollösung (in Acetonitril) hinzugefügt hat, lässt man das Reaktionsgemisch für 2 h bei Raumtemperatur rühren und verfolgt den Reaktionsfortschritt mittels DC. Nach dem Einengen der Mischung am Rotationsverdampfer erhält man nach der säulenchromatographischen Reinigung 3'-Phosphoramidit-2'-Perfluoro-Uridin. Diese Nukleotide sind Ausgangspunkt für das das Phosphoramiditverfahren, in welchem die monomeren Nukleotide miteinander gekoppelt werden. Hierbei werden monomere Nukleotidbausteine als 3'-Phosphoramidite an einer Festphase miteinander verknüpft. Der Aufbau der Sequenz erfolgt vom 3'- zum 5'-Ende, wobei bei Standardsynthesen ein *"leadernucleosid*" über ein 3'-Succinat mit einer geeigneten Festphase verknüpft ist. Die als 3'-Phosphoramidit eingebrachten Nukleoside tragen an der 5'-Hydroxylgruppe eine säurelabile Schutzgruppe und an den exocyclischen Aminofunktionen der Basen basenlabile Schutzgruppen. Der immer neu zu wiederholende Synthesezyklus umfasst folgende Reaktionen: Phosphoramidit wird durch die Zugabe einer schwachen Säure, wie 4,5-Dicyanoimidazol oder 1-H-Tetrazol, aktiviert und an die 5'-Hydroxygruppe des immobilisierten Nukleosides gekoppelt. Die nicht umgesetzten OH-Gruppen werden mit Essigsäureanhydrid acetyliert und somit für weitere Umsetzungen blockiert. Die Oxidation erfolgt mit wässriger Jodlösung und führt zum Phosphorsäuretriester. Die säurelabile Tritylschutzgruppe wird mit einer Dichloressigsäurelösung entfernt. Nach vollendeter Sequenz werden das Oligonukleotid und die Schutzgruppen der Nukleobasen durch Behandlung mit wässrigem Ammoniak bei 55°C abgespalten.

Das Phosphoramiditverfahren oder andere chemische Verfahren zur Herstellung von Oligomeren und Nukleinsäuren werden zum gegenwärtigen Stand der Technik bereits von diversen Firmen weltweit kommerziell angeboten und die erzielbare Länge der Nukleotidsequenzen hat die Größe von künstlichen Genen erreicht.

### Ausführungsbeispiel 8: alternative Synthese von perfluorierten Oligonukleotiden und Nukleinsäuren

Es ist auch die Perfluorierung ganzer Oligomere und Nukleinsäuren möglich, beispielsweise durch säurekatalysierte Verfahren mit Fluorsäure oder die Polymersisation von perfluorierten Nukleotiden durch Polymerasen (Polymerase-Ketten-Reaktion) möglich.

### Ausführungsbeispiel 9: Perfluorierung von modifizierten Nukleinsäurebausteinen

Nukleinsäuren können zur Stabilisierung oder Eliminierung der elektrischen Ladung derivatisiert werden, beispielsweise durch Phosphorothioate, elektrisch neutrale Methylphosphonat-Derivate, elektrisch neutrale Dimethylsulfon-Derivate oder Derivatisierung am 2'-Kohlenstoffatom der Ribose. Die Perfluorierungsmöglichkeiten liegen ebenso wie bei den normalen Nukleinsäureausteinen am modifizierten Zucker-Phosphat-Rückgrat und an den Basen. Perfluorierungswege siehe Synthese von perfluorierten Nukleosiden, Nukleotiden und perfluorierten Oligonukleotiden.

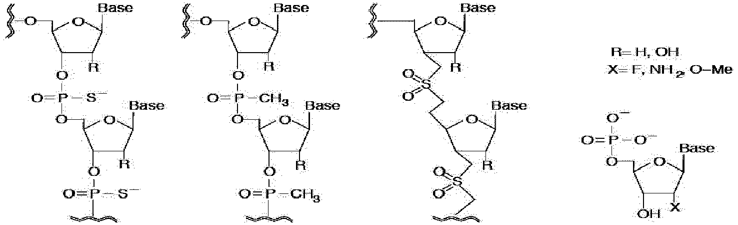

### Ausführungsbeispiel 10: Synthese von perfluorierten Peptidnukleinsäure-Monomeren in Form von Alanyl-Nukleoaminosäuren

Peptidnukleinsäure-Monomere sind Nukleotid-Analoge, bei denen das Ribose-Phosphodiesterrückgrat durch ein peptidisches, achirales Rückgrat ersetzt wurde, das auf N-(2-Aminoethyl)glycin-Untereinheiten oder anderen Peptideinheiten basiert. Die perfluorierten Basen sind dabei über eine Carboxymethylen-Einheit mit dem Rückgrat verknüpft. Während die Perfluorierung an den NH₂-Gruppen der Nukleobasen keinen Einfluss auf den Einbau in ein Oligomer zeigt, wirkt die Perfluorierung an den aminoterminalen und carboxyterminalen Enden der Peptideinheiten syntheseabbrechend. Die Synthese von Alanyl-Nukleoaminosäuren geht von *N*-Boc-L-Serin und *N*-Boc-D-Serin aus, die in *N*Boc-L-Serinlacton bzw. *N*-Boc-D-Serinlacton überführt wurden. Das Boc-Serinlacton reagiert mittels nukleophiler Ringöffnung durch das Benzyloxycarbonyl-geschützte Cytosin und dem Guanin-Vorläufer 2-Amino-6-chlorpurin zu Boc-L-AlaG-OH bzw. Boc-D-AlaG-OH und Boc-AlaCZ-OH bzw. seinem Enantiomer. Die Schutzgruppe Benzyloxycarbonyl an der exozyklischen Aminofunktion des Cytosins ist für die nachfolgende Peptid Festphasensynthese notwendig. Das Guanin benötigt keine Schützung, da die exozyklische Aminogruppe eine äußerst geringe Nukleophilie aufweist.

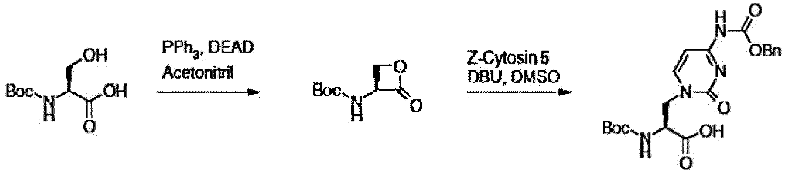

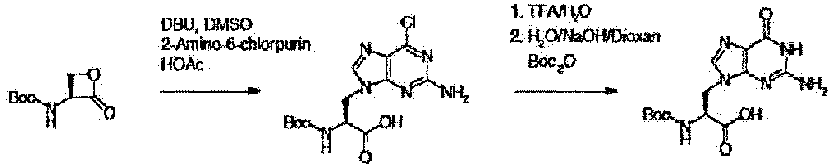

Als Ausgangsverbindung zur Synthese der Homoalanyl-Nukleoaminosäuren diente N-Boc-L-Asparaginsäurebenzylester bzw. Boc-D-Asparaginsäurebenzylester. Die Seitenketten wurden mit BH3·THF zum Alkohol reduziert und über eine Appel-Reaktion zum *N*-Boc-L-γ-Brom-homoalanylbenzylester bzw. *N*-Boc-D-γ-Brom-homoalanylbenzylester bromiert.

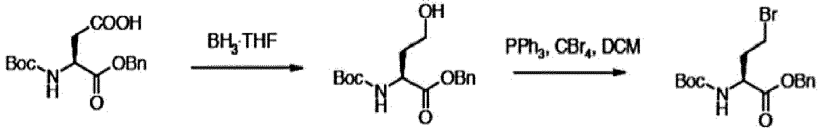

Unter K₂CO₃ wurde eine Nukleophile Substitution des Bromids mit Benzyloxycarbonylgeschütztem Cytosin und 2-Amino-6-chlorpurin durchgeführt. Im nächsten Schritt wurde TFA/H₂O hydrolisiert, wobei gleichzeitig die Boc-Schutzgruppe entfernt wurde. Der darauf folgende Schritt wurde hydrogenolytisch mit PdO·H₂O durchgeführt. Anschließend wurde mit Boc-Anhydrid zum Boc-L-HalG-OH bzw. Boc-D-HalG-OH geschützt.

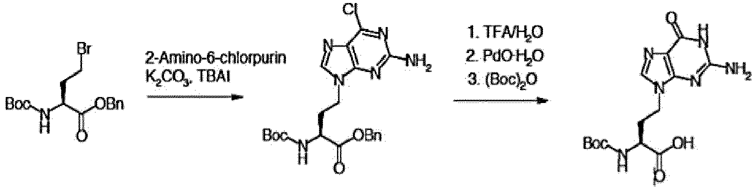

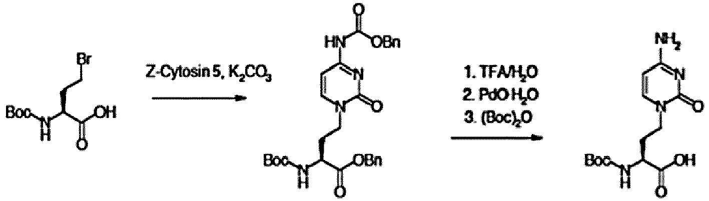

Die Perfluorierung der Monomere erfolgte über eine Williamsonsche Ethersynthese mittels K₂CO₃/Aceton und einem funktionalisierten Perfluorocarbon-Molekül über 48 Stunden. Dabei wurden alle zugänglichen NH₂- und OH-Gruppen an den Nukleobasen und an den Peptidbausteinen perfluoriert. Beispielhaft für diese Perfluorierung ist hier die Reaktion mit C₈F₁₇J dargestellt.

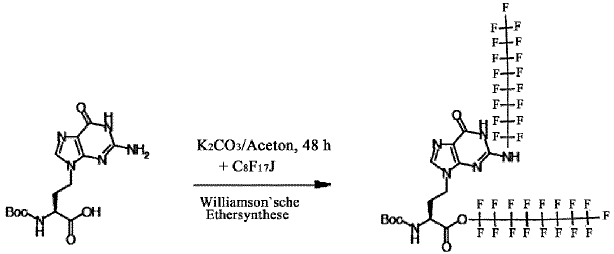

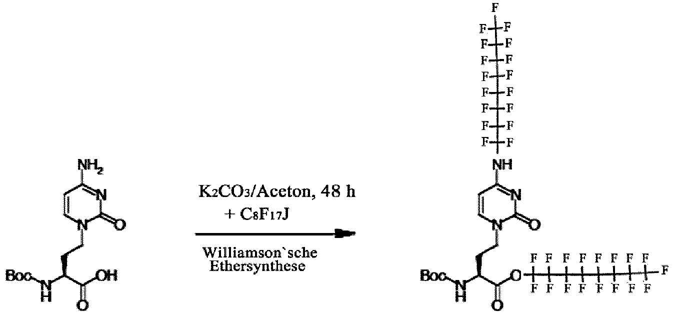

Eine weitere Möglichkeit ist die Maskierung der OH-Gruppen des Peptidanteils, bevor eine Perfluorierung stattfindet. Unter diesen Umständen werden nur die NH₂-Gruppen der Nukleobasen perfluoriert. Hierzu müssen die Hydroxy-Gruppen mit Ditertbutylsilylditriflat geschützt werden. Nach der Perfluorierung müssen diese OH-Gruppen wieder entschützt werden, um frei zu sein für die Peptidsynthese. Mit diesen Monomeren ist eine Oligomer-Synthese möglich, in der bereits perfluorierte Monomere eingebaut werden. Aufgrund der zusätzlich erforderlichen Reaktionsschritte erscheint es aber einfacher, zunächst das Oligomer zu synthetisieren und dann zu perfluorieren.

### Weitere beispielhaft ausgeführte Perfluorierungen:

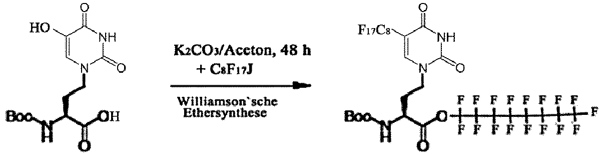

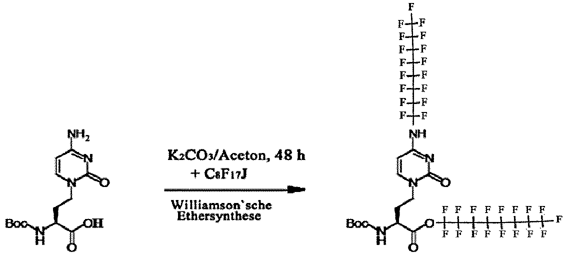

### Ausführungsbeispiel 11: Synthese von perfluorierten Peptidnukleinsäure-Oligomeren und Peptidnukleinsäuren

Bei Peptidnukleinsäuren wurde das vollständige Ribose-Phosphodiesterrückgrat durch ein peptidisches, achirales Rückgrat ersetzt, das auf N-(2-Aminoethyl)glycin-Untereinheiten oder anderen Peptideinheiten basiert. Die perfluorierten Basen sind dabei über eine Carboxymethylen-Einheit mit dem Rückgrat verknüpft. Zunächst wurden die Nukleobasen mit den Peptid-Einheiten verknüpft. Anschliessend wurden die Monomere in einer Festphasensynthese zu Oligomeren verknüpft. Es hat sich vereinfachend erwiesen, die Perfluorieungsschritte erst nach der Synthese der Oligomeren durchzuführen. Die Perfluorierungsschritte folgen den Reaktionsschritten bei der Perfluorierung der Monomere.

Die Synthese von Alanyl-Nukleoaminosäuren geht von *N*-Boc-L-Serin und *N*-Boc-D-Serin aus, die in *N*Boc-L-Serinlacton bzw. *N*-Boc-D-Serinlacton überführt wurden. Das Boc-Serinlacton reagiert mittels nukleophiler Ringöffnung durch das Benzyloxycarbonyl-geschützte Cytosin und dem Guanin-Vorläufer 2-Amino-6-chlorpurin zu Boc-L-AlaG-OH bzw. Boc-D-AlaG-OH und Boc-AlaCZ-OH bzw. seinem Enantiomer. Die Schutzgruppe Benzyloxycarbonyl an der exozyklischen Aminofunktion des Cytosins ist für die nachfolgende Peptid Festphasensynthese notwendig. Das Guanin benötigt keine Schützung, da die exozyklische Aminogruppe eine äußerst geringe Nukleophilie aufweist.

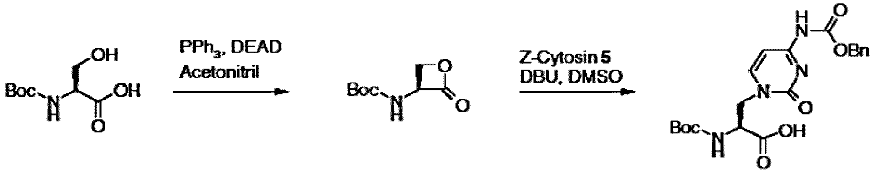

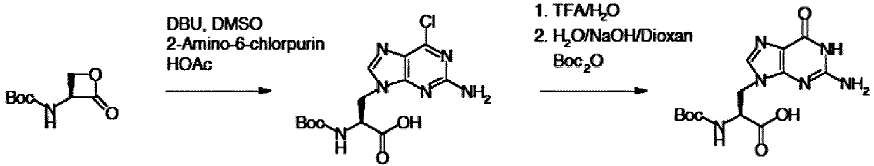

Als Ausgangsverbindung zur Synthese der Homoalanyl-Nukleoaminosäuren diente N-Boc-L-Asparaginsäurebenzylester bzw. Boc-D-Asparaginsäurebenzylester. Die Seitenketten wurden mit BH3·THF zum Alkohol reduziert und über eine Appel-Reaktion zum *N*-Boc-L-γ-Brom-homoalanylbenzylester bzw. *N*-Boc-D-γ-Brom-homoalanylbenzylester bromiert.

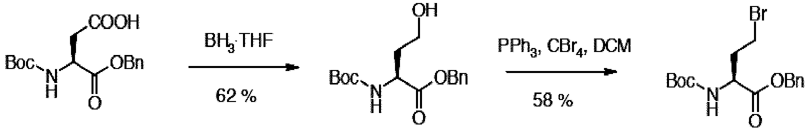

Unter K2CO3 wurde eine Nukleophile Substitution des Bromids mit Benzyloxycarbonylgeschütztem Cytosin und 2-Amino-6-chlorpurin durchgeführt. Im nächsten Schritt wurde TFA/H2O hydrolisiert, wobei gleichzeitig die Boc-Schutzgruppe entfernt wurde. Der darauf folgende Schritt wurde hydrogenolytisch mit PdO·H2O durchgeführt. Anschließend wurde mit Boc-Anhydrid zum Boc-L-HalG-OH bzw. Boc-D-HalG-OH geschützt.

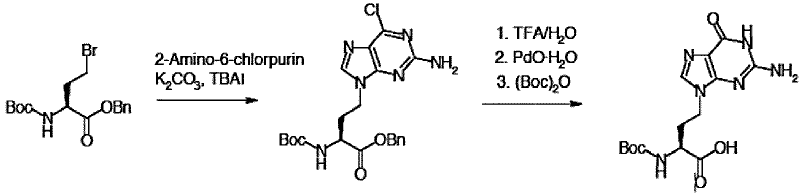

Da der Erhalt der Benzyloxycarbonyl-Schutzgruppe der Cytosinbase erwünscht war, konnte hier keine hydrogenolytische Spaltung der Benzylgruppe erfolgen. Es wurde daher eine basische Verseifung mit NaOH/Dioxan/H2O durchgeführt.

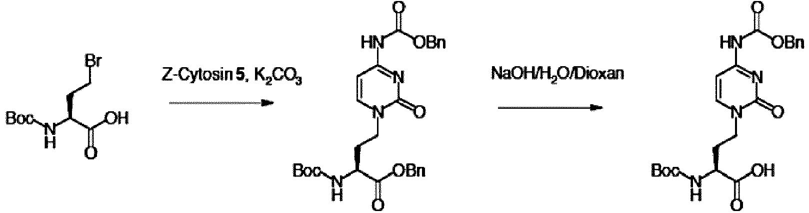

Die Synthese von Peptidnukleinsäuren wird analog zur Synthese von Peptiden durchgeführt. Die Synthese erfolgt an Festphasensystemen. Die Synthese kann entweder durch Boc-Syntheseverfahren oder durch das Fmoc-Syntheseverfahren durchgeführt werden. Hier wurde das Boc-Syntheseverfahren gewählt:
Als Kupplungsreagenzien wurden für die Aminosäuren HBTU (*N*-(1*H*-Benzotriazol-1-yl)-1,1,3,3-tetramethyluronium-hexafluorophosphat) und HOBt (1-Hydroxybenzotriazol) eingesetzt. Die Homoalanyl-Nukleoaminosäuren wurden mit HATU und HOAt aktiviert. Als feste Phase wurde MBHA-PS-Harz, belegt mit Boc-L-Lys(2-CI-Z)-OH, verwendet. Je nach Aminosäure wurde zwischen 35 Minuten und zwei Stunden gekuppelt. Die Schutzgruppen der Seitenketten wurden so gewählt, dass sie gleichzeitig mit der sauren Abspaltung vom Harz entfernt werden konnten. Für Lysin wurde die Seitenkette mit (2-Cl-Z), für Glutaminsäure mit (OBn) und für Tyrosin mit (2-Br-Z)-Gruppe geschützt.
- 1. Entschützung: 5 % *m*-Kresol inTFA (1 x 5 min, 1 x 10min); 2. Waschen: DCM/NMP (5 x) + Pyridin;
- 1. Kupplung: 5.0 eq. Boc-Hal-OH bzw. 5.0 eq. Boc-AS-OH, 4.5 eq. HATU bzw. HBTU, 5.0 eq. HOAt bzw. HOBt 12 eq. DIPEA, NMP; 2. Waschen: DCM/NMP (5 x), 10 % Piperidin in NMP (3 x), DCM/NMP (5 x);
- 1. Capping: Ac2O/DIPEA/NMP (1:1:8), (2 x 5 min); 2. Waschen: DCM/NMP (5 x), 10 % Piperidin in NMP (3 x), DCM/NMP (5 x); Abspaltung: TFA/TFMSA/*m*-Kresol (8:1:1)

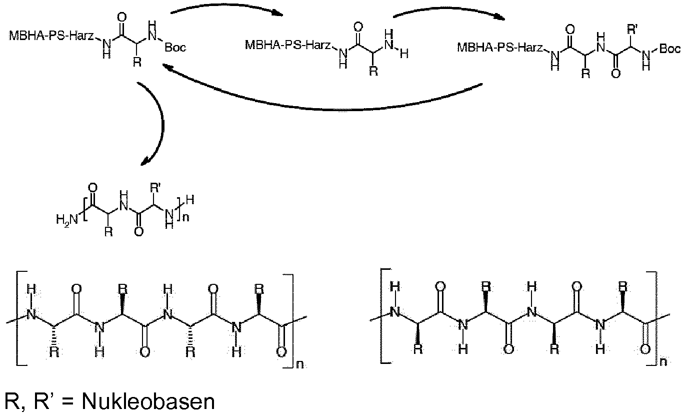

Nach dieser Methode wurde ein 15mer-Peptid mit inkorporierten Nukleoaminosäuren hergestellt.

Anschließend erfolgte eine Williamsonsche Ethersynthese mittels K₂CO₃/Aceton und einem funktionalisierten Perfluorocarbon-Molekül für 48 Stunden. Die Perfluorierung erreichte alle zugänglichen NH₂- und OH-Gruppen, wobei die Reaktion analog zur Perfluorierung einzelner Nukleobasen und Peptidnukleinsäure-Monomere (wie oben beschrieben) stattfand. Durch Variierung der Reaktionszeit (2 h bis 72 h) konnte der Perfluorierungsgrad verringert bzw. gesteigert werden.

### Ausführungsbeispiel 12: Auswahl der Sollbruchstellen

Hier genutzt wurden folgende geeignete Sollbruchstellen: Die fPFC/mRNA-Komplexe werden durch Endocytose aufgenommen und in Lysosomen verpackt. Dabei erfolgt ein deutlicher pH-Sprung von 7,4 bis 7,2 im extrazellulären Raum auf bis zu 4,0 im Lysosom, der durch eine ATP-abhängige Protonenpumpe verursacht wird (Serresi et al. 2009). Dieser niedrige pH-Wert von 4,0 ist für die Wahl der Sollbruchstelle entscheidend. Es gibt eine Reihe säurelabiler Sollbruchstellen, die für das fPFC-System in Frage kommen (Warnecke, 2008, Warnecke 2010). Aber auch glycosidische Bindungen an der 2'-Position hydrolysieren bei niedrigen pH-Werten, siehe Schema 14.

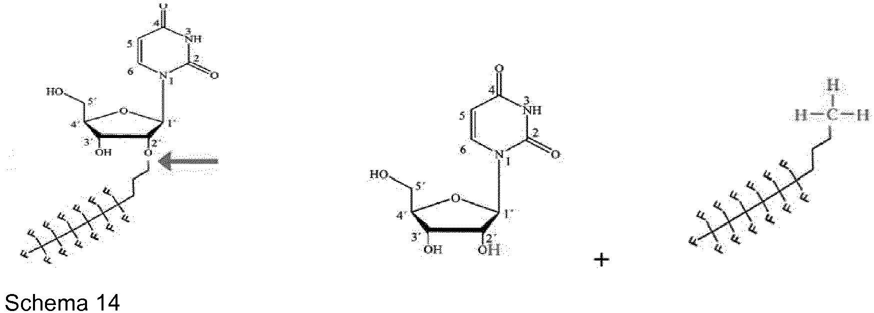

Da Perfluorkohlenwasserstoffe die Moleküle vor Hydrolasen maskieren, kommt es zur chemischen Hydrolyse, so dass die Anbindung von schädlichen OH-Gruppen an die Perfluorkohlenwasserstoffkette verhindert wird. So bleibt die abgespaltene Perfluorkohlenwasserstoffkette innert und geht keine Verbindung mit Zellmolekülen ein. Nutzbar sind aber auch Sollbruchstellen, die im Cytoplasma brechen (ca. pH-Werten =7).

### Ausführungsbeispiel 13: Herstellung einer Emulsion

A) Für die Einstellung bestimmter Partikelgrößen kann es notwendig, sein Tenside zu verwenden. Hier verwendet wurde Pluronic F-68. In 10 ml destilliertem Wasser werden 5 mg Pluronic F-68 gelöst. Dazu werden 0,5 ml einer funktionalisierten Perfluorocarbon/mRNA-Lösung (1,0g/1,0 Microliter) gegeben. Anschließend wird sonifiziert, 3 Zyclen, Stärke 60. Die enthaltene Emulsion wird anschließend zentrifugiert (1200U/5min), um die zu großen Partikel abzusetzen. Im Überstand darüber befinden sich Partikel mit einer Partikelgröße von 50-100 Nanometern. Diese werden genutzt.
B) Lösungsmittelemulsion: In 2 ml Tetrahydrofuran werden 0,5 ml einer funktionalisierten Perfluorocarbon/mRNA-Lösung (1,0g/1,0 Microliter) gegeben und darin gelöst. Anschließend wird auf 10 ml mit destilliertem Wasser aufgefüllt. Die enthaltene Emulsion wird anschließend zentrifugiert (1200U/5min), um die zu großen Partikel abzusetzen. Im Überstand darüber befinden sich Partikel mit einer Partikelgröße von 50-100 Nanometern. Diese werden genutzt.
C) In 10 ml destilliertem Wasser werden 0,5 ml einer funktionalisierten Perfluorocarbon/mRNA-Lösung (1,0g/1,0 Microliter) gegeben. Anschließend wird sonifiziert, 3 Zyclen, Stärke 60. Die enthaltene Emulsion wird anschließend zentrifugiert (1200U/5min), um die zu großen Partikel abzusetzen. Im Überstand darüber befinden sich Partikel mit einer Partikelgröße von 50-100 Nanometern. Diese werden genutzt.

### Ausführungsbeispiel 14:

Herstellung einer künstlichen mRNA mit therapeutischer Sequenz zur Behandlung einer erworbenen genetischen Erkrankung
Die Herstellung einer künstlichen perfluorierten mRNA erfolgt wie oben beschrieben. Die Sollbruchstelle dieses Systems ist eine glycosidische Bindung. Zusätzlich wird bei gleicher Codierungsinformation der künstlichen mRNA deren GC-Gehalt erhöht, um die Lebensdauer (Resistenz gegenüber RNAsen) zu steigern.

Die Komplexe aus künstlicher mRNA und funktionalisiertem Perfluorocarbon besitzen sowohl hydrophile als auch hydrophobe Eigenschaften und benötigen kein weiteres Tensid, Herstellung der Emulsion erfolgt wie unter C) des Ausführungsbeispiels 13 beschrieben. Die in einem Puffer aufbereitete Emulsion wird durch eine Apparatur zu einem Aerosol verarbeitet, das als Inhalationsspray appliziert wird und über die Lunge in den Blutkreislauf des Körpers gelangt. Der Komplex zirkuliert im Blut und wird unspezifisch durch Endocytose/Pinocytose in die Körperzellen aufgenommen. In den Endosomen und Lysosomen der Zelle kommt es zum Bruch der Sollbruchstellen durch chemische Hydrolyse. Die freigesetzte mRNA und die freigesetzten Perfluorocarbon-Moleküle werden vom Endosom oder vom Lysosom ins Cytoplasma freigesetzt. Im Cytoplasma kommt es zur Translation der mRNA und zur Bildung der therapeutischen Proteine. Das Transportsystem (Perfluorocarbon-Moleküle) ist inert und kann nicht mit Zellmolekülen reagieren. Aufgrund seines Dampfdrucks und anderer physikalischer Eigenschaften wird das Transportsystem über Lungen- und Nierenfunktion ausgeschieden.

### Ausführungsbeispiel 15:

Einschleusen einer therapeutischen siRNA in der Krebstherapie:
Herstellung der siRNA und Anbinden von Transferrin an das System erfolgt wie beschrieben; ebenso die Herstellung der Emulsion. Diese Emulsion wird intravenös verabreicht. Durch den Liganden Transferin werden die Partikel besonders stark in Tumorzellen aufgenommen, wodurch die Therapie mit siRNA in spezifischen Zielzellen wirken kann. In den Endosomen oder den Lysosomen werden durch chemische Hydrolyse die Sollbruchstellen gebrochen. Es kommt zur Freisetzung von siRNA und Perfluorocarbon-Molekülen ins Cytoplasma. Die Perfluorocarbon-Moleküle werden über Lungen- und Nierenfunktion ausgeschieden.

**Ausführungsbeispiel 16**: Eine therapeutische Impfung gegen HPV Typ 16 siRNA zur Stilllegung einer spezifischen Genexpression von HPV Typ 16 und deren Emulsion wird wie oben beschrieben hergestellt. Diese Emulsion wird in einem Puffer zu einer Tinktur verarbeitet, die auf die Schleimhaut appliziert wird. Die Komplexe dringen in das Gewebe ein und werden von den Zellen durch Endocytose/Pinocytose aufgenommen. Wirkungsweg der siRNA und Auscheidungsweg der Perfluorocarbon-Moleküle sind wie oben beschrieben.

### Ausführungsbeispiel 17:

Nachweis der Aufnahme der perfluorierten Nukleinsäuren in die Zelle
Um den Weg der perfluorierten Nukleinsäuren in die Zelle zu verfolgen, wurde das folgende Konstrukt mit Rhodamin-Anbindung verwendet:

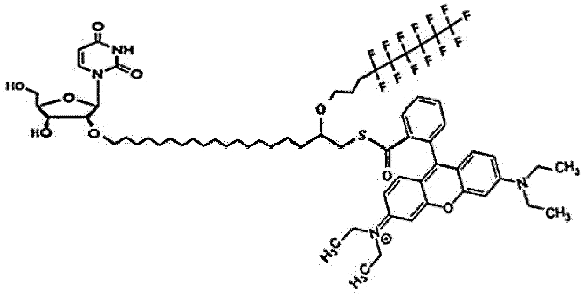

Die Verbindung wurde zunächst in Tetrahydrofuran (THF) gelöst und diese Lösung anschließend in Isopropanol titriert. Die dadurch erzielten Partikel hatten eine durchschnittliche Größe von 50 nm.

Als Zellkultur wurden Zellen der Linie HEK 293 verwendet. Die Transfektion erfolgte einen Tag nach der Zellverbreiterung. Als Kontrolle wurde eine Transfektion mit reinen Rhodamin-Partikeln äquivalenter Rhodamin-Konzentration zur Probe der perfluorierten Nukleinsäuren durchgeführt. Unmittelbar nach der Transfektion erschien das Zellkulturmedium der Zellen mit perfluorierten Nukleinsäuren klar und unverändert. Das Medium mit Rhodamin erschien leicht trüb und rot gefärbt. Eine Erklärung hierfür ist womöglich, dass das Rhodamin bei den perfluorierten Nukleinsäuren am PFC-Partikel gebunden ist und als kleiner Partikel zu Boden sinkt, während im Medium mit reinem Rhodamin der Farbstoff vollständig gelöst ist.

20 Minuten nach der Transfekton konnte man bei den Zellen mit perfluorierten Nukleinsäuren eine homogene Färbung gleichmässig über die Zelloberfläche verteilt finden. Bei den Zellen mit reinem Rhodamin war zu diesem Zeitpunkt keine definierte Färbung zu erkennen.

24 Stunden nach der Transfektion zeigte sich die Färbung der Zellen mit perfluorierten Nukleinsäuren verglichen zum Zeitpunkt nach 20 Minuten verändert: Die gleichmäßig homogene Färbung auf der Zelloberfläche konnte nicht mehr beobachtet werden. Stattdessen zeigte sich eine granuläre Färbung, deren Vesikel in der Färbungsintensität erhöht waren, wohingegen die Zwischenräume kaum eine Färbung zeigten.

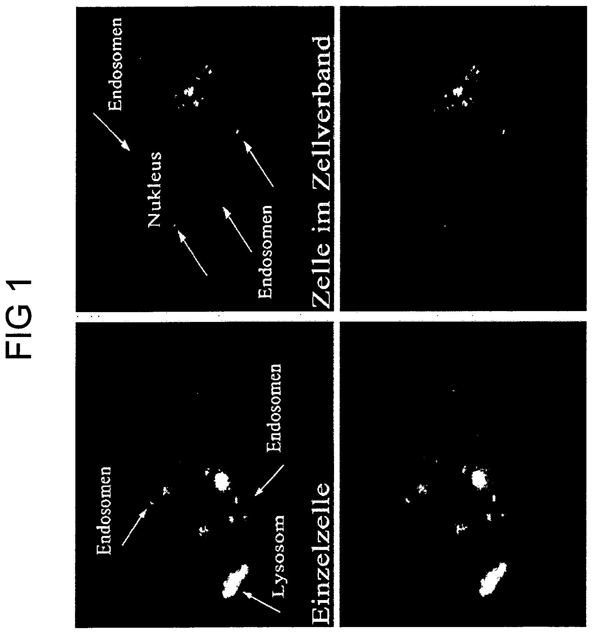

Bei Untersuchung dieses Vorgangs durch Aufnahmen am konfokalen Mikroskop (siehe Figur 1) zeigte sich, das die perfluorierten Nukleinsäuren in Endosomen und Lysosomen transportiert wurden. Die mit perfluorierten Nukleinsäuren gefüllten Endosomen und Lysosomen konnten im gesamten Zytoplasma der Zelle nachgewiesen werden. Nicht zu finden waren die Partikel im Nukleus (Kernlokalisationssequenz oder Zellteilung erforderlich). Zusätzlich konnte man erkennen, dass sich im Zytoplasma bereits ein Betrag an aus den Endosomen/Lysosomen freigesetzten Partikeln befand, was in der diffusen Färbung außerhalb der Vesikel zu erkennen war.

Im Gegensatz dazu konnte bei der Transfektion mit reinem Rhodamin zwar nach 24 Stunden ebenfalls eine Färbung beobachtet werden, diese war aber vergleichsweise wesentlich schwächer und diffus auf der Zelloberfläche statt im Cytoplasma und granulär.

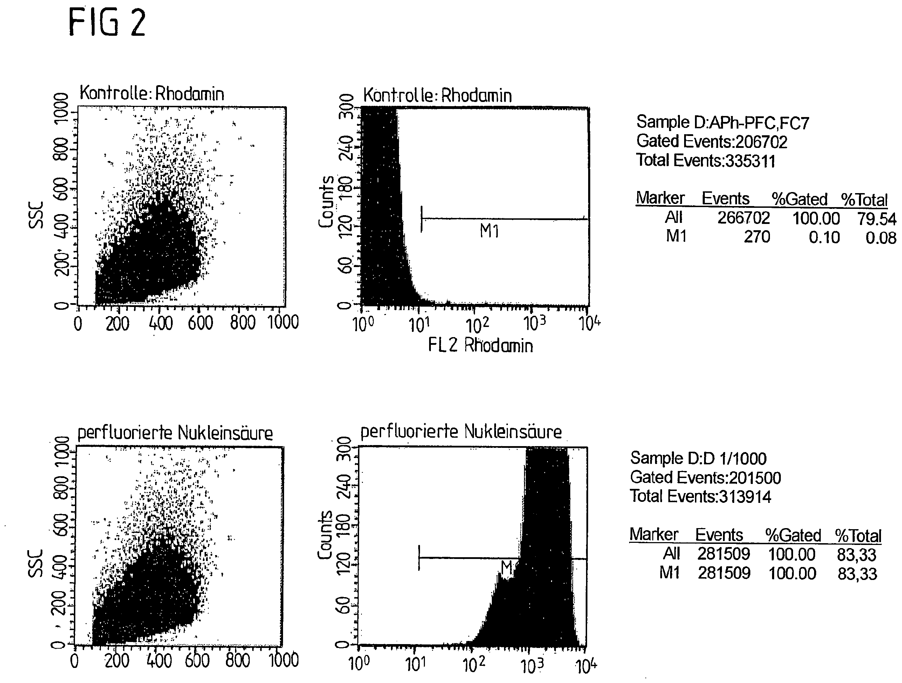

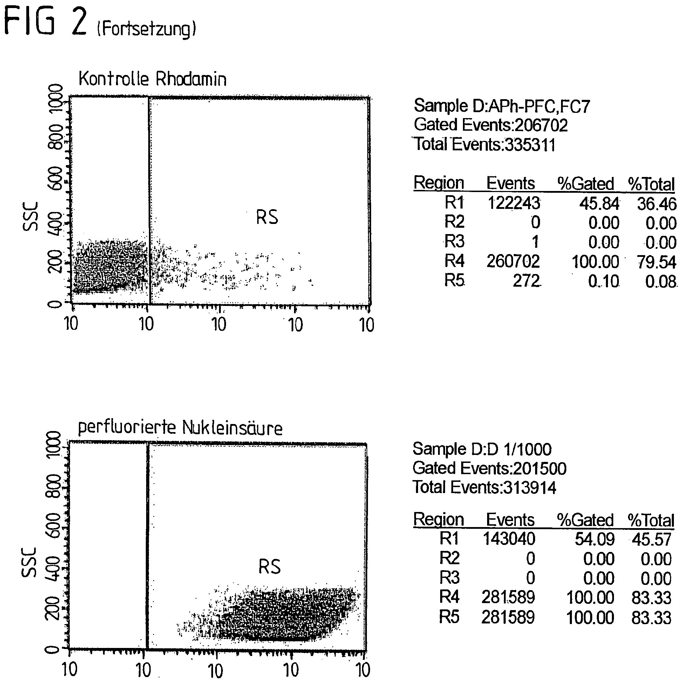

Die transfizierten Zellen wurden zusätzlich am FACS (Fluorescence activated cell sorting) untersucht (siehe Figur 2). Die FACS-Untersuchungen wurden an mit reinem Rhodamin (Kontrolle) und mit perfluorierten Nukleinsäuren transfizierten Zellen durchgeführt, wobei die Ergebnisse der konfokalen Mikroskopie bestätigt wurden. Perfluorierte Nukleinsäuren werden effektiv von der Zelle aufgenommen, in Endosomen und Lysosomen verpackt und von diesen ins Zytoplasma freigesetzt.

Durch die hohe Effektivität der Aufnahme der perfluorierten Nukleinsäuren in die Zelle stellt diese Methode eine echte Alternative zum viral vermittelten Gentransfer und zu anderen Transfermethoden von Nukleinsäuren und deren Analogen (modifizierte Nukleinsäuren, Peptid-Nukleinsäuren) in die Zelle dar.

## Patentansprüche

1. Verbindung der allgemeinen Formel (XIX)
A - B - C(F', G')- B- E (XIX)
oder der allgemeinen Formel (XXII)
A - B - C(F', G')- E (XXII)
wobei
- A ein Perfluorocarbon (PFC) ist,
- B mindestens eine Sollbruchstelle ist,
- C abwesend oder ein Linker ist, wobei der Linker ausgewählt ist aus der Gruppe bestehend aus: geradlinige oder verzweigte acyclische oder cyclische, polycyclische oder heterocyclische aliphatische Alkane, Alkene, Alkine, aromatische Verbindungen,
- E ein Oligonukleotid oder eine Nukleinsäure ist,
- F' abwesend oder ein Ligand ist, wobei der Ligand ausgewählt ist aus der Gruppe bestehend aus: Transferrin, Folsäure, Galactose, Lactose, Mannose, Epidermal Growth Factor, RGD-Peptide, Biotin oder einer Kernlokalisationssequenz,
- G' abwesend oder ein Marker ist, wobei der Marker einen Fluoreszenz-Farbstoff ist,
wobei:
- A, B, C, E, F',G' über kovalente Bindungen verknüpft sind, und
- A-B, optional mit C, F' und/oder G', an E an der 2'- Position der Ribose, an der Phosphatgruppe oder an der Nukleobase verknüpft ist, wobei:
A ausgewählt ist aus der Gruppe der Perfluorkohlenwasserstoffe (PFC) bestehend aus C₄-C₃₀, bevorzugt C₄-C₂₀, Alkane, Alkene oder Alkine, die linear oder verzweigt sein können, bei denen alle H-Atome durch F-Atome ersetzt sind,
und wobei:
die Sollbruchstelle B in Form einer glykosidischen Bindung, einer Disulfidbrücke, einer Estergruppe, Ethergruppe, Peptidbindung, Imin-Bindung, Hydrazon-Bindung, Acylhydrazon-Bindung, Ketalbindung, Acetalbindung, cis-Aconitrilbindung, Tritylbindung, beta-D-Glucosylceramid und/oder Dithiothreitol, oder in Form eines Plasmalogenperfluorids, einer Vinylethergruppe oder Orthoesters ausgebildet ist.

2. Verbindung nach einem der vorhergehenden Ansprüche, **dadurch gekennzeichnet, dass** E Nukleotide umfasst, welche Nukleoside umfassen, wobei die Nukleoside ausgewählt sind aus der Gruppe enthaltend: Adenosin, Guanosin, Cytidin, 5-Methyluridin, Uridin, Desoxyadenosin, Desoxyguanosin, Thymidin, Desoxyuridin, Desoxycytidin oder modifizierte Nukleoside wie 2-Thiocytidin, N4-Acetylcytidin, 2'-O-Methylcytidin, 3-Methylcytidin, 5-Methylcytidin, 2-Thiouridin, 4-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxycarbonylmethyl-Uridin, 5-Methoxyuridin, 2'-O-Methyluridin, Ribothymidin, 1-Methyladenosin, 2-Methyladenosin, N6-Methyladenosin, N6-Isopentenyladenosin, 2'-O-Methyladenosin, Inosin, 1-Methylinosin, 1-Methylguanosin, N2-2-Methylguanosin, N2-2,2-Dimethylguanosin, 7+-Methylguanosin, 2'-O-Methylguanosin, Queuosin , β-D-Galactosyl-queuosin, β-D-Mannosyl-queuosin, Archaeosin, 2'-O-Ribosyladenosinphosphat, N6-Threonylcarbamoyladenosin, Lysidin, Nicotinsäure, Riboflavin und Pantothensäure, NADPH, NADH, FAD, Coenzym A, und Succinyl-Coenzym A, Puromycin, Aciclovir, Ganciclovir und deren "Spiegelmere".

3. Verbindung nach einem der vorhergehenden Ansprüche, **dadurch gekennzeichnet, dass** E ausgewählt ist aus der Gruppe der einzelsträngigen oder doppelsträngigen Oligonukleotide und Nukleinsäuren, wobei diese eine Länge von 2 Basenpaaren bis größer als 1.000.000 Bp aufweisen, wobei folgende Längenbereiche jeweils bevorzugt sind: 10 bis 50 bp, 15 bis 25 bp, 25 bis 200 bp, 25 bis 100 bp, 200 bis 300 bp, 200 bis 500 bp, 500 bis 1500 bp, 800 bis 1300 bp, 1500 bis 20.000 bp, 1500 bis 5000 bp, 3000 bis 8000 bp, 20.000 bis 1.000.000 bp oder 20.000 bis 50.000.

4. Verbindung nach einem der vorhergehenden Ansprüche zur Verwendung als Medikament zum nicht-viralen Transfer von mindestens einem Molekül E in mindestens eine Zelle eines Menschen.

5. In vitro Verwendung einer Verbindung nach einem der vorhergehenden Ansprüche zum nicht-viralen Transfer von mindestens einem Molekül E in mindestens eine Zelle eines eukaryotischen Organismus, insbesondere von Tieren oder Menschen.

6. Pharmazeutische Zusammensetzung umfassend mindestens eine Verbindung nach einem der Ansprüche 1 bis 4 und mindestens eine oberflächenaktive Substanz.

7. Pharmazeutische Zusammensetzung nach Anspruch 6, **dadurch gekennzeichnet, dass** die Zusammensetzung in Form einer Dispersion, Suspension, Emulsion oder Lösung insbesondere mit einer mittleren Partikelgröße zwischen 2 nm und 200 µm, bevorzugt zwischen 50 nm und 400 nm, insbesondere bevorzugt bei 50 nm, vorliegt.

## Claims

1. A compound of general formula (XIX)
A - B - C (F', G') - B - E (XIX)
or general formula (XXII)
A - B - C (F', G') - E (XXII)
wherein
- A is a perfluorocarbon (PFC),
- B is at least one predetermined breaking point,
- C is absent or is a linker, wherein the linker is selected from the group consisting of: linear or branched acyclic or cyclic, polycyclic or heterocyclic aliphatic alkanes, alkenes, alkynes, aromatic compounds,
- E is an oligonucleotide or a nucleic acid,
- F' is absent or is a ligand, wherein the ligand is selected from the group consisting of: transferrin, folic acid, galactose, lactose, mannose, epidermal growth factor, RGD peptide, biotin or a nuclear localization signal,
- G' is absent or is a marker, wherein the marker is a fluorescent dye,
wherein:
- A, B, C, E, F', G' are linked via covalent bonds, and
- A-B, optionally with C, F' and/or G', is linked to E at the 2'-position of ribose, to the phosphate group or to the nucleobase,
wherein:
A is selected from the group of perfluorocarbons (PFC) consisting of C₄-C₃₀, preferably of C₄-C₂₀,
alkanes, alkenes or alkynes, which may be linear or branched, in which all H atoms are replaced by F atoms, and wherein:
the predetermined breaking point B is structured in the form of a glycoside bond, a disulfide bridge, an ester group, ether group, peptide bond, imine bond, hydrazone bond, acylhydrazone bond, ketal bond, acetal bond, cis-aconitrile bond, trityl bond, beta-D-glucosylceramide and/or dithiothreitol, or in the form of a plasmalogen perfluoride, a vinyl ether group or ortho ester.

2. The compound according to one of the preceding claims, **characterized in that** E comprises nucleotides comprising nucleosides, wherein the nucleosides are selected from the group containing: adenosine, guanosine, cytidine, 5-methyluridine, uridine, deoxyadenosine, deoxyguanosine, thymidine, deoxyuridine, deoxycytidine or modified nucleosides such as 2-thiocytidine, N4-acetylcyditine, 2'-O-methylcytidine, 3-methylcytidine, 5-methylcytidine, 2-thiouridine, 4-thiouridine, pseudouridine, dihydrouridine, 5-(carboxyhydroxymethyl)-uridine, 5-carboxymethylaminomethyl-uridine, 5-methylaminomethyl-uridine, 5-methoxy-carbonylmethyl-uridine, 5-methoxyuridine, 2'-O-methyluridine, ribothymidine, 1-methyladenosine, 2-methyladenosine, N6-methyladenosine, N6-isopentenyladenosine, 2'-O-methyladenosine, inosine, 1-methylinosine, 1-methylguanosine, N2-2-methylguanosine, N2-2,2-dimethylguanosine, 7+-methylguanosine, 2'-O-methylguanosine, queuosine, β-D-galactosyl-queuosine, β-D-mannosyl-queuosine, archaeosine, 2'-O-ribosyladenosine phosphate, N6-threonylcarbamoyladenosine, lysidine, nicotinic acid, riboflavin and pantothenic acid, NADPH, NADH, FAD, coenzyme A, and succinyl-coenzyme A, puromycin, aciclovir, ganciclovir and their "spiegelmers."

3. The compound according to one of the preceding claims, **characterized in that** E is selected from the group of single-stranded or double-stranded oligonucleotides and nucleic acids, wherein these have a length of from 2 base pairs to more than 1,000,000 Bp, wherein the following length ranges are respectively preferred: 10 to 50 bp, 15 to 25 bp, 25 to 200 bp, 25 to 100 bp, 200 to 300 bp, 200 to 500 bp, 500 to 1500 bp, 800 to 1300 bp, 1500 to 20,000 bp, 1500 to 5000 bp, 3000 to 8000 bp, 20,000 to 1,000,000 bp or 20,000 to 50,000.

4. The compound according to one of the preceding claims for use as a medication for non-viral transfer of at least one molecule E into at least one cell of a human being.

5. An in vitro use of a compound according to one of the preceding claims for non-viral transfer of at least one molecule E into at least one cell of an eukaryotic organism, especially of animals or humans.

6. A pharmaceutical composition comprising at least one compound according to one of claims 1 to 4 and at least one surface-active substance.

7. The pharmaceutical composition according to claim 6, **characterized in that** the composition exists in the form of a dispersion, suspension, emulsion or solution, especially with an average particle size between 2 nm and 200 µm, preferably between 50 nm and 400 nm, particularly preferably 50 nm.

## Revendications

1. Composé répondant à la formule générale (XIX)
A - B - C (F', G') - B - E (XIX)
ou à la formule générale (XXII)
A - B - C (F', G') - E (XXII)
dans lequel
- A est un perfluorocarbure (PFC),
- B est au moins un point destiné à la rupture,
- C est absent ou un lieur, dans lequel le lieur est sélectionné à partir du groupe constitué par : des alcanes, alcènes, alcynes, composés aromatiques acycliques ou cycliques, polycycliques ou hétérocycliques linéaires ou ramifiés,
- E est un oligonucléotide ou un acide nucléique,
- F' est absent ou un ligand, dans lequel le ligand est sélectionné à partir du groupe constitué par : la transferrine, l'acide folique, le galactose, le lactose, le mannose, le facteur de croissance de l'épiderme, les peptides RGD, la biotine ou une séquence de localisation nucléaire,
- G' est absent ou un marqueur, dans lequel le marqueur est un colorant fluorescent,
dans lequel :
- A, B, C, E, F', G' sont liés par l'intermédiaire de liaisons covalentes, et
- A - B, éventuellement avec C, F' et/ou G', est lié au niveau de E en position 2' du ribose, au niveau du groupe phosphate ou de la base nucléaire,
dans lequel :
A est sélectionné à partir du groupe des perfluorocarbures (PFC) constitué par des alcanes, des alcènes ou des alcynes en C₄ à C₃₀, de préférence de C₄ à C₂₀, qui peuvent être linéaires ou ramifiés, dans lesquels tous les atomes de H sont substitués par des atomes de F,
et dans lequel :
le point destiné à la rupture B est réalisé sous la forme d'une liaison glycosidique, d'un pont disulfure, d'un groupe ester, d'un groupe éther, d'une liaison peptidique, d'une liaison imine, d'une liaison hydrazone, d'une liaison acylhydrazone, d'une liaison cétal, d'une liaison acétal, d'une liaison cis-aconitrile, d'une liaison trityle, d'un bêta-D-glucosylcéramide et/ou d'un dithiothréitol, ou sous la forme d'un perfluorure de plasmalogène, d'un groupe éther vinylique ou d'un orthoester.

2. Composé selon l'une des revendications précédentes, **caractérisé en ce que** E comprend des nucléotides, lesquels comprennent des nucléosides, dans lequel les nucléosides sont sélectionnés à partir du groupe contenant : l'adénosine, la guanosine, la cytidine, la 5-méthyluridine, l'uridine, la désoxyadénosine, la désoxyguanosine, la thymidine, la désoxyuridine, la désoxycytidine ou des nucléosides modifiés tels que la 2-thiocytidine, la N4-acétylcytidine, la 2'-O-méthylcytidine, la 3-méthylcytidine, la 5-méthylcytidine, la 2-thiouridine, la 4-thiouridine, la pseudouridine, la dihydrouridine, la 5-(carboxyhydoxyméthyl)-uridine, la 5-carboxyméthylaminométhyl-uridine, la 5-méthylaminométhyl-uridine, la 5-méthoxycarbonylméthyl-uridine, la 5-méthoxy-uridine, la 2'-O-méthyluridine, la ribothymidine, la 1-méthyladénosine, la 2-méthyladénosine, la N6-méthyladénosine, la N6-isopentényladénosine, la 2'-O-méthyladénosine, l'inosine, la 1-méthylinosine, la 1-méthylguanosine, la N2-2-méthylguanosine, la N2-2,2-diméthylguanosine, la 7+-méthylguanosine, la 2'-O-méthylguanosine, la queuosine, la β-D-galactosyl-queuosine, la β-D-mannosyl-queuosine, l'archaéosine, le 2'-O-ribosyl-adénosine-phosphate, la N6-thréonylcarbamoyladénosine, la lysidine, l'acide nicotinique, la riboflavine et l'acide pantothénique, le NADPH, le NADH, le FAD, la coenzyme A et la succinyl-coenzyme A, la puromycine, l'aciclovir, la ganciclovir et leurs « spiegelmères ».

3. Composé selon l'une des revendications précédentes, **caractérisé en ce que** E est sélectionné à partir du groupe des oligonucléotides et des acides nucléiques simple brin ou double brin, dans lequel ceux-ci présentent une longueur de 2 paires de bases jusqu'à plus de 1 000 000 paires de bases, dans lequel les plages de longueurs suivantes sont respectivement de préférence : 10 à 50 pb, 15 à 25 pb, 25 à 200 pb, 25 à 100 pb, 200 à 300 pb, 200 à 500 pb, 500 à 1 500 pb, 800 à 1 300 pb, 1 500 à 20 000 pb, 1 500 à 5 000 pb, 3 000 à 8 000 pb, 20 000 à 1 000 000 pb ou 20 000 à 50 000.

4. Composé selon l'une des revendications précédentes pour une utilisation en tant que médicament pour le transfert non viral d'au moins une molécule E dans au moins une cellule d'un humain.

5. Utilisation in vitro d'un composé selon l'une des revendications précédentes pour le transfert non viral d'au moins une molécule E dans au moins une cellule d'un organisme eucaryote, en particulier d'animaux ou d'humains.

6. Composition pharmaceutique comprenant au moins un composé selon l'une des revendications 1 à 4 et au moins une substance tensioactive.

7. Composition pharmaceutique selon la revendication 6, **caractérisée en ce que** la composition se présente sous la forme d'une dispersion, d'une suspension, d'une émulsion ou d'une solution en particulier avec une taille de particules moyenne entre 2 nm et 200 µm, de préférence entre 50 nm et 400 nm, plus préférablement de 50 nm.
